# EUROPEAN PATENT APPLICATION

(11) **EP 4 574 216 A2**
(43) Date of publication of application: **25.06.2025**
(21) Application number: 25174217.7
(22) Date of filing: 24.03.2023
(51) Int. Cl.: A61Q 5/12

(54) **COMPOSITION FOR ENHANCING KERATIN FIBERS**

(30) Priority: 25.03.2022 US 202263323608 P
(62) Divisional of application: 23715783.9
(71) Applicant: Wella Germany GmbH, 64295 Darmstadt (DE)
(72) Inventor: Springob, Christian, 64295 Darmstadt (DE); Riemann, Ingo, 64295 Darmstadt (DE)

(57) **Abstract**

The present invention is directed to a hair treatment composition and method for its use. Embodiments of the hair treatment composition are a stand-alone conditioner, a stand-alone leave-on conditioner, a stand-alone rinse-off conditioner, and a stand-alone mask which include at least a primary component comprising a dicarboxylic acid optionally substituted with one or more hydroxyls and a saturated or unsaturated, linear organic fatty acid. An optional secondary component comprising at least one of a fatty acid ester, a fatty alcohol ester, a cationic surfactant, a nonionic surfactant, a cationic polymer, a fatty alcohol, a preformed polymer, a coloring agent, a rheology control agent, a suspending agent, a fatty hydrocarbon and one or more common conditioning additives may be included.

## Description

### REFERENCE TO RELATED APPLICATIONS

This application claims priority to US Provisional Patent Application U.S. Serial Number 63/323,608 filed March 25, 2022.

### FIELD OF THE INVENTION

The present invention relates to a hair treatment composition comprising components providing rehabilitation of damaged keratin fibers, and a method for application of the composition.

### BACKGROUND OF THE INVENTION

Keratin fibers and human hair in particular are damaged by daily routines such as brushing and combing, external influences like sun light and pollution and oxidative and/or reductive treatments like hair coloration, bleaching or perming. The damage caused by these activities includes removal of the F-layer fatty acids, breakage of disulfide links of keratin protein and erosion of the keratin protein materials at the surfaces of the hair fiber cuticles. The damage tends to roughen the hair fiber surfaces much like a rasp roughens wood. The roughened hair strands lack smoothness, tend to misbehave and are unruly. Hair care goals have involved attempts to reverse these tendencies.

One such attempt has involved hair care and repair agents such as hair cortex repair formulations and associated methods. It is thought that damage to the hair cortex can result in unravelling cortex protein bundles and can lead to the unruliness and roughness. One possible answer has involved use of cysteine thiol binding with maleate molecules to re-bond cleaved disulfide links in hair cortex. See International Patent Application WO 2015/017768 A1. Another possible answer has involved a method for restructuring of hair fiber cortex with cysteine and at least one dicarboxylic acid. See WO 2005/115314 A1.

However, these methods have been only partially successful. They do not address cuticle erosion, subsurface and surface degradation of keratin protein. Therefore, there is still a need to provide compositions delivering superior hair cuticle repair as this remains one of the key hair concerns of consumers. A further need is the improvement of the integrity and the healthiness of hair and deliver shine, suppleness, a smooth hair feel and good disentangling properties.

### SUMMARY OF THE INVENTION

The present invention is directed a hair treatment composition and corresponding method for treatment of the cuticle of keratin fibers, specifically cuticles of anagenic human hair damaged by daily routine and chemical treatment. Embodiments of the hair treatment composition deliver components to protect and repair the damaged cuticles of keratin fibers, such as anagenic hair strands of humans. The compositional components enable repair of broken keratin protein inner connections, smoothing of hair strand roughness, lubrication of hair strands and melding of split strand ends, in other words, effective, significant control of the frizzing of hair. The compositional components deliver hair strand treatment without causing heavy weighting of hair strands, oiliness. Additionally, the present invention provides improved anti-frizz, improved shine, improved dry feel and prevention of the feeling of greasy, uncleaned hair.

Compositional aspects of the invention are directed to embodiments of a hair treatment composition comprising a general stand-alone hair conditioner, a stand-alone rinse-off conditioner, a stand-alone leave-on conditioner, or a stand-alone mask. These embodiments are designed to deliver anti-frizz, shine, suppleness, smoothness, disentanglement, minimal or no oiliness, minimal or no heavy weighting to the hair.

Accordingly, these embodiments of the hair treatment composition comprise:
A. a general stand-alone conditioner, or
B. a stand-alone rinse-off conditioner, or
C. a stand-alone leave-on conditioner, or
D. a stand-alone mask;
wherein the conditioner A), B), C) or D) comprises at least an aqueous or aqueous-organic medium with an acidic primary component and an optional lipophilic secondary component.

The primary component of conditioner A), B), C) or D) comprises at least a dicarboxylic acid and a fatty monocarboxylic acid wherein
1) the dicarboxylic acid comprises:
   a) a C3-C6 saturated, linear dicarboxylic acid with one hydroxyl group or a salt thereof; or
   b) a C3-C8 saturated, linear dicarboxylic acid with two or three hydroxyl groups or a salt thereof; or
   c) a C2 - C13 saturated, linear dicarboxylic acid optionally substituted with one or two or three or more hydroxyl groups or a salt thereof; or
   d) any combination of a), b) and c); and,
2) the fatty monocarboxylic acid comprises:
   a) a C16-C18 linear, mono-unsaturated fatty monocarboxylic acid or salt thereof; or
   b) a C10 - C30 linear mono-unsaturated fatty acid or salt thereof, or
   c) a C10 - C30 linear saturated or mono- or di-unsaturated fatty monocarboxylic acid; or
   d) any combination of a), b) and c).
Preferably, the dicarboxylic acid of the primary component comprises:
a) a C3-C6 saturated, linear dicarboxylic acid with one hydroxyl group or a salt thereof; or
b) a C3-C8 saturated, linear dicarboxylic acid with two or three hydroxyl groups or a salt thereof.
Preferably, the fatty monocarboxylic acid of the primary component comprises:
a) a C16-C18 linear, mono-unsaturated fatty monocarboxylic acid or salt thereof;

The optional lipophilic secondary component of conditioner A), B), C) or D) comprises at least a lipophilic fatty acid ester and a lipophilic hydrocarbon wherein
1) the lipophilic fatty acid ester comprises:
   a) a linear unsaturated C10 - C30 fatty acid with 1or 2 unsaturations esterified with C1-C10 mono, di or tri alcohol; or,
   b) a linear saturated C10 - C30 fatty acid esterified with C1-C10 mono, di or tri alcohol; and
2) the lipophilic hydrocarbon comprises a saturated or unsaturated, linear or branched C10 to C40 hydrocarbon.
Preferably, the lipophilic fatty acid ester comprises a linear monounsaturated C10 to C30 fatty acid monoesterified with a C3-C6 triol.
Preferably, the lipophilic hydrocarbon comprises a C28-C30 branched hydrocarbon that is saturated or has from 3 to 6 unsaturations.

The embodiments of the hair treatment composition may optionally be combined with additives for the secondary component including but not limited to a saturated or unsaturated C10 - C30 fatty alcohol, a C2-C6 mono or di carboxylic acid ester of the fatty alcohol, a lipid hydrocarbon mimic, a surfactant additive, a film forming polymer, a rheologic agent, a preformed polymer, a color agent, a micro-particulate and other conditioning additives. Some additional secondary component additives may include at least one cationic surfactant such as but not limited to a quaternary ammonium compound formed of at least one saturated and/or unsaturated fatty C10 -C30 amine quaternized with one to three C1-C3 alkyl groups. Included also are at least one non-ionic surfactant such as a polyalkoxy-fatty alcohol ether, a fatty acid glycoside ester and/or a fatty acid carbohydrate (saccharide) ester. Included also are at least one cationic polymer based on at least one olefinic, ester, amide, urethane and/or urea polymer, a cellulose and/or polysaccharide derivative substituted by multiple tertiary amine or quaternary ammonium groups. Further additives comprise a thickener, a strengthening agent for providing hair strand body, a silicone, a silica, diatomaceous earth, a clay such as a kaolin and smectite phyllosilicate, a coloring agent and/or a shine agent. Further additives typically present in conditioner compositions, and which may be included in the hair treatment composition according to the invention, include an antioxidant, a preservative, an anti-microbial, a UV protectant, fragrance, a humectant and/or an emollients.

According to another aspect of the invention, hair, optionally wetted or damped before treatment, may be conditioned by treatment of the hair with any of the embodiments of the hair treatment composition, and may then be optionally rinsed and/or dried after application. Dry hair may alternatively be treated with the stand-alone mask followed by a comb out and/or an optional light to moderate rinsing after a sufficient time for dry conditioning.

A further aspect of the invention is directed to a cleaning and conditioning regimen. This regimen for cleaning and conditioning hair comprises a first step of cleaning hair with a readily available cleaning composition such as but not limited to a sulfate anionic surfactant shampoo or sulfate-free anionic and/or nonionic surfactant shampoo followed by a second step of conditioning the hair with the general stand-alone hair conditioner A), the stand-alone leave-on conditioner B), or the stand-alone rinse-off conditioner C) of the hair treatment composition. Use of the mask D) typically is practiced without a prior cleansing and cleaning step although the mask D) may be used after the hair has been cleansed, cleaned and dried. The regimen may be practiced through use of a kit comprising a unit containing a cleansing and cleaning composition containing a detergent and other constituents for cleaning hair and a unit containing any of the stand-alone conditioner embodiments of the hair treatment composition.

### DEFINITIONS

Unless defined otherwise, all technical and scientific terms used herein have the same meaning as commonly understood by a person of ordinary skill in the art.

As used in the specification and the appended statements, the singular forms "a," "an" and "the" include plural referents unless the context clearly dictates otherwise.

The term "may" in the context of this application means "is permitted to" or "is able to" and is a synonym for the term "can." The term "may" as used herein does not mean possibility or chance.

The term and/or in the context of this application means one or the other or both. For example, an aqueous solution of A and/or B means an aqueous solution of A alone, an aqueous solution of B alone and an aqueous solution of a combination of A and B.

The term "about" is understood to mean ±10 percent of the recited number, numbers or range of numbers.

The term "about 0 wt%" is understood to mean that no substance, compound or material to which zero (0) refers is present, up to a negligible but detectable amount is present, assuming that the detectability can be determined on a parts per million basis.

Reference throughout this specification to "embodiment", "one embodiment" or "preferred embodiment" or "more preferred embodiment" or "most preferred embodiment" means a feature of any one or more elements and can be used in connect with different elements of the invention. A particular feature, structure, or characteristic described in connection with an embodiment may not be the same as another feature, structure or characteristic of another embodiment of the presently claimed invention. Thus, appearances of the phrases "in one embodiment" or "an embodiment" or "in a preferred embodiment" in various places throughout this specification are not necessarily all referring to the same embodiment but may refer to different embodiments of the presently claimed invention. Furthermore, the features, structures, or characteristics in one or more embodiments may be combined in any suitable manner, as would be apparent to a person skilled in the art from this disclosure. Furthermore, while some embodiments described herein include some, but not other features included in other embodiments, combinations of features of different embodiments are meant to be within the scope of the subject matter, and form different embodiments, as would be understood by those in the art. For example, in the appended claims, any of the claimed embodiments can be used in any combination.

The term "user" or "customer" means the person preparing the hair treatment composition. The user is for example a professional hair stylist working in a salon and is different from the subject on whose hair the composition is applied, or the user is identical to the person on whose hair the composition is applied.

The term "fatty" in the context of an organic compound means an alkyl or alkenyl group or an alkane or alkene of at least eight carbons, may extend to about sixty carbons, preferably from about ten to about thirty carbons and may be linear or branched with from one to eight **C1-**C6 alkyl groups. The branched alkyl groups count as extra carbons relative to the number of carbons of the primary chain of the fatty alkyl or alkenyl group or alkane or alkene. The number of olefinic unsaturations of the fatty alkenyl or alkene may range from 1 to 8 unsaturation sites. The unsaturation sites may be unconjugated, partially conjugated or fully conjugated. This definition of "fatty" is understood to describe all fatty alkyl or alkenyl groups or alkanes and alkenes described herein. **In** instances where one or more recitations of carbon number, branching and unsaturation are included with specific instances of fatty alkyl or alkenyl groups or alkanes or alkenes, those specific recitations apply.

An alkyl or alkenyl group, an alkane or alkene or an organic compound containing an alkyl or alkenyl group which is not characterized as "fatty" in the context of this invention may have an alkyl or alkenyl group carbon count of from one to about 10 carbons, preferably from one to about 8 carbons, and the alkyl or alkenyl group may be linear or branched.

The terms "unsaturated" and "unsaturation" in the context of an organic compound mean at least one olefinic double bond and may include multiple instances of olefinic double bonds up to about 8 instances of olefinic unsaturation.

The term "stand-alone" in the context of a conditioner or mask means that the conditioner or mask is formulated and is to be used separate and apart from any other composition or formulation developed for use on hair such as but not limited to shampoo. Thus, the general stand-alone conditioner, stand-alone leave-on conditioner, stand-alone rinse-off conditioner and/or stand-alone mask are not components of a shampoo, a hair cleansing composition, a hair oxidative dye composition, a hair bleach composition and/or other compositions for use on hair. These conditioner embodiments deliver conditioning properties including but not limited to smoothing, softening and conditioning hair to deliver shine, suppleness, smoothness, disentanglement, free-flowing, fluffiness and ease of combing as well as lack of oiliness, lack of heavy weighting to the hair.

The term "leave-on" in the context of a conditioner means that the constituents of the conditioner are formulated so that application of the conditioner delivers the above-described conditioning properties to the hair while not overly wetting the hair. The leave-on conditioner is formulated to have a variable viscosity depending upon whether the leave-on conditioner is formulated as an aerosol, a non-aerosol spray, a lotion, a crème, mousse, gel or paste. These formulations may range from fluid like water to thick like a gel or mousse by adjusting the weight percentage concentrations of constituents and medium. Thorough distribution and coverage of the conditioner constituents throughout the hair may be accomplished by rubbing, massaging, painting, finger dabbing and/or similar means. The medium formulation is adjusted to enable its evaporation by brushing and/or combing the hair. The leave-on stand-alone conditioner according to the invention may optionally be formulated with lower amounts of highly hydrophobic (lipophilic) lipid constituents such as hydrocarbons and fatty acid esters as well as little or no anionic surfactants and moderate to slightly higher amounts of moderately hydrophobic lipid constituents such as fatty alcohols and cationic surfactants as well as nonionic surfactants relative to the general stand-alone conditioner and the rinse-off stand-alone conditioner.

The terms "rinse-off" and "general" in the context of a conditioner mean that the weight percentages of constituents of the conditioner are formulated to deliver a variable viscosity depending on whether the conditioner is formulated as a spray, a crème, a lotion, a gel, a mousse, a paste or a similar flowable to gel-like constitution, which may but not necessarily be thixotropic. The rinse-off and general conditioners can be applied to wet hair after shampooing and the so-treated hair may be rinsed with room temperature to warm water. The general conditioner according to the invention may be optionally formulated with related concentrations of anionic and cationic surfactants that can form coacervates during rinsing. The rinsing removes excess conditioner while leaving at least a portion of the conditioner components such as but not limited to coacervates on the hair. The rinse-off and general conditioners according to the invention may optionally be formulated with higher amounts of highly hydrophobic lipid constituents such as hydrocarbons and fatty acid esters and with higher amounts of moderately hydrophobic lipid constituents such as fatty alcohols and cationic surfactants as well as nonionic surfactants.

The term "mask" in the context of the invention means that components of the hair treatment composition are formulated to provide crème, paste or gel-like constitution with higher concentrations of moisturizers, humectants, nutrients, the primary component and the optional secondary component and a low to moderate concentration of medium relative to the concentrations of components of the leave-on and rinse-off conditioner embodiments of the hair treatment composition. A mask is typically a leave-on embodiment and may optionally and briefly be rinsed after remaining on the hair for a reasonable period of time.

Where features or aspects of the invention are described in terms of Markush groups, those skilled in the art will recognize that the invention is also thereby described in terms of any individual member or subgroup of members of the Markush group. For example, if X is described as selected from the group consisting of methyl, ethyl or propyl, claims or embodiment statements for X being methyl and claims or embodiment statements for X being ethyl and X being propyl are fully described. Moreover, where features or aspects of the invention are described in terms of Markush groups, those skilled in the art will recognize that the invention is also thereby described in terms of any combination of individual members or subgroups of members of Markush groups. Thus, for example, if X is described as selected from the group consisting of bromine, chlorine, and iodine, and Y is described as selected from the group consisting of methyl, ethyl, and propyl, claims or embodiment statements for X being bromine and Y being methyl are fully described.

If a value of a variable that is necessarily an integer, e.g., the number of carbon atoms in an alkyl group or the number of substituents on a ring, is described as a range, e.g., 0-4, what is meant is that the value can be any integer between 0 and 4 inclusive, i.e., 0, 1, 2, 3, or 4. Similarly, values expressed in a range format should be interpreted in a flexible manner to include not only the numerical values explicitly recited as the limits of the range, but also to include all the individual numerical values or sub-ranges encompassed within that range as if each numerical value and sub-range were explicitly recited. For example, a range of "about 0.1% to about 5%" or "about 0.1% to 5%" should be interpreted to include not just about 0.1% to about 5%, but also the individual values (e.g., 1%, 2%, 3%, and 4%) and the sub-ranges (e.g., 0.1% to 0.5%, 1.1% to 2.2%, 3.3% to 4.4%) within the indicated range.

The term "anagenic hair" as used herein means hair strands that are in direct connection with a hair follicle which is in either the anagen or telogen state. Anagenic hair is present in one of these states on the head of a person, a human, and its follicle is appurtenant to a sebum gland that substantially continuously secretes sebum and long chain fatty acids onto the surfaces of the hair shaft. As the hair grows from the follicle, it carries with it coatings of secreted sebum and long chain fatty acids. The fatty acid coating is known as the f layer and is tightly entwined with the keratin protein at the hair shaft surface. Hair cut from a living person is no longer anagenic hair.

Keratin fibers means hair strands on the scalp of a person, preferably anagenic hair. Keratin fibers, aka hair strands, are comprised of cells forming an inner cortex as the core and a cuticle as the outer covering surrounding the cuticle. The hair strand chemical components include proteins, poly-nucleic acids, amino acids, minerals, melanin, lipids including fatty acids, fatty alcohols, triglycerides, phospholipids, cholesterol, and squalene. These components are configured as cells, fibrils, connective tissue, extracellular links, pigments, keratin and associated structures. The outer layer of the cuticle is configured as a sheath of overlapping flat cells having a structure like the bark of a palm tree or scales of a fish. Like the scales of a fish, the surface of the cuticle is smooth in the direction from the root to the tip and rough in the direction from the tip to the root.

### DETAILED DESCRIPTION

The present invention is directed to a hair treatment composition that delivers components and substances for replenishment of, and addition to, damaged anagenic hair. The hair treatment composition constitutes a stand-alone aspect meaning that it is applied to hair by itself uncombined with any other applique formulation, cleaning formulation or other treatment. This aspect of the hair treatment composition comprises embodiments of the hair treatment composition comprising at least the primary component described above, wherein the embodiments are:
1) a general stand-alone conditioner, and/or
2) a stand-alone leave-on conditioner, and/or
3) a stand-alone rinse-off conditioner, and/or
4) a stand-alone mask.

The hair treatment composition may also be incorporated as a second part of a kit for a cleaning and conditioning regimen. The kit includes as a first unit, a composition for cleansing and cleaning of the hair with a detergent-like formulation such as a known shampoo or a brush-on-brush-off cleaning formulation, and as a second unit, one or more of the embodiments of the hair treatment composition described above. The cleaning and conditioning regimen comprises cleansing and cleaning the hair using the first unit and conditioning the hair using the second unit.

### The Primary Component

The hair treatment composition embodiments described above comprise the medium and at least the primary component comprising the dual organic acid constituents of a C2-C13 dicarboxylic acid and a C10-C30 fatty monocarboxylic acid and/or salts thereof in which:
a) the C2-C13 dicarboxylic acid may be saturated, may be linear and may optionally but preferably include 1 or more hydroxyl groups; and;
b) the fatty C10-C30 linear monocarboxylic acid may be saturated or unsaturated and preferably may be unsaturated with one or more unsaturations, preferably one unsaturation.
It is believed that these dicarboxylic acid and the fatty monocarboxylic acid constituents may respectively develop hydrophilic and lipophilic properties of the hair treatment composition that at least in part develop its healing and anti-frizz characteristics for hair.

Preferrable embodiments of the saturated dicarboxylic acid may comprise a linear saturated C2-C13 dicarboxylic acid optionally with 1, 2, 3, 4, 5, 6 or more hydroxyl groups depending upon the number of non-carboxylic acid carbons present in the dicarboxylic acid.

More preferable embodiments are the linear C3- C13 dicarboxylic acid optionally substituted by one or more hydroxyl groups.

Even more preferably, the embodiments of the saturated dicarboxylic acid may comprise a linear C3-C8 saturated dicarboxylic acid with 1, 2, 3 or 4 hydroxyl groups depending upon the number of non-carboxylic acid carbons present in this preferred dicarboxylic acid.

Especially more preferred embodiments of the saturated dicarboxylic acid may comprise a C3-C6 linear dicarboxylic acid substituted with 1 or 2 hydroxyls, especially with 1 hydroxyl.

The dicarboxylic acid may also be any combination of the foregoing dicarboxylic acid embodiments, preferred embodiments and more preferred and especially more preferred embodiments.

Exemplary individual saturated C2-C13 dicarboxylic acids include any one or more of or any combination of oxalic acid, malonic acid, succinic acid, glutaric acid, adipic acid, heptanedioic acid, octanedioic acid, nonanedioic acid, decanedioic acid, brassylic acid (C13 saturated dicarboxylic acid).

Exemplary individual saturated C3-C13 dicarboxylic acids with 1 or more hydroxyls include any one or more of, or any combination of, malic acid, hydroxysuccinic acid, tartaric acid, hydroxyglutaric acid, , trihydroxyglutaric acid, hydroxyadipic acid, galactaric acid, hydroxyheptanedioic acid, hydroxyoctanedioic acid, hydroxydecanedioic acid.

Preferred exemplary individual saturated C3-C13 dicarboxylic acids include any one or more of or any combination of, malonic acid, succinic acid, glutaric acid, adipic acid and/or brassylic acid.

Preferred exemplary individual saturated C3-C6 dicarboxylic acids with 1 or more hydroxyls include malic acid, tartaric acid, hydroxyglutaric acid and/or galactaric acid.

More preferred exemplary individual saturated dicarboxylic acids with 1 or more hydroxyls include malic acid, tartaric acid and any mixture thereof with malic acid being especially more preferred.

The primary component of hair treatment composition also comprises the fatty C10-C30 saturated or unsaturated, linear mono carboxylic acid. Embodiments of the fatty C10-C30 saturated monocarboxylic acid may be linear with the carboxylic acid terminating the linear chain.

Preferred embodiments of the fatty saturated monocarboxylic acid include the fatty C10 - C30 linear monocarboxylic acid with the carboxyl group at the terminus.

Preferred embodiments of fatty unsaturated monocarboxylic acid include the fatty C10 - C30 linear monocarboxylic acid with the carboxyl group at the terminus and 1 olefinic unsaturation.

More preferred embodiments of the fatty unsaturated monocarboxylic acid include the fatty C14-C22 linear monocarboxylic acid with the carboxyl at the terminus and 1 olefinic unsaturation, especially more preferably a C16-C18 linear unsaturated fatty monocarboxylic acid.

The fatty monocarboxylic acid may also be any combination of the foregoing fatty monocarboxylic acid embodiments, preferred embodiments and more preferred embodiments.

Exemplary individual saturated fatty monocarboxylic acids include any one or more of or any combination of caprylic acid, lauric acid, myristic acid, palmitic acid, stearic acid, arachidic acid, behenic acid, lignoceric acid, cerotic acid.

Exemplary individual unsaturated fatty monocarboxylic acids include myristoleic acid, palmitoleic acid, sapienic acid, oleic acid, elaidic acid, vaccenic acid, linoleic acid, linoelaidic acid, linolenic acid, arachidonic acid, eicosapentaenoic acid, erucic acid.

Preferred exemplary individual saturated fatty monocarboxylic acids include myristic acid, palmitic acid, stearic acid.

Preferred exemplary individual unsaturated fatty monocarboxylic acids include myristoleic acid, palmitoleic acid (C16, cis-9), sapienic acid (C16 cis-6), oleic acid, elaidic acid, vaccenic acid, linoleic acid, linoelaidic acid and erucic acid.

More preferred exemplary individual unsaturated fatty acids include palmitoleic acid, sapienic acid, oleic acid, elaidic acid, vaccenic acid, linoleic acid and linelaidic acid.

Especially more preferred exemplary individual unsaturated fatty acids include palmitoleic, sapienic, oleic and elaidic acids.

Most preferred exemplary individual unsaturated fatty acids include palmitoleic/sapienic and oleic acids with oleic acid being the most especially preferred example.

The primary component of the hair treatment composition comprises a combination of the dicarboxylic acid and the fatty monocarboxylic acid. Any combination of any of the above-described exemplary individual dicarboxylic acids and any of the above-described exemplary individual fatty monocarboxylic acids provides the primary component of the hair treatment composition. The combinations may include:
a) any of the exemplary individual dicarboxylic acids with any of the exemplary individual monocarboxylic acids described above;
b) any of the exemplary individual dicarboxylic acids with any of the preferred or more preferred individual exemplary fatty monocarboxylic acids;
c) any of the preferred or more preferred or especially more preferred exemplary individual dicarboxylic acids with any of the exemplary individual fatty monocarboxylic acids.
d) any of the preferred exemplary individual dicarboxylic acids with any of the preferred or more preferred or most preferred exemplary individual fatty monocarboxylic acids;
e) any of the preferred or more preferred or especially more preferred exemplary individual dicarboxylic acids with any of the exemplary individual preferred fatty monocarboxylic acids;
f) any of the more preferred or especially more preferred exemplary individual dicarboxylic acids with any of the more preferred or especially more preferred or most preferred exemplary individual fatty monocarboxylic acids;
g) Any of the above- described combinations a) through f) in which the dicarboxylic acid is substituted by one or more hydroxyls and the fatty carboxylic acid is unsaturated.

Exemplary preferred combinations of the dicarboxylic acid and the fatty monocarboxylic acid include the saturated dicarboxylic acids of 3 to 6 carbon chain count, especially those also having a hydroxyl substitution and the unsaturated fatty monocarboxylic acids of 14 to 18 carbon chain count.

Preferred exemplary combinations of the saturated dicarboxylic acid and the fatty monocarboxylic acid comprise succinic acid, glutaric acid or adipic acid with an unsaturated fatty acid such as palmitoleic acid, sapienic acid, oleic acid, elaidic acid or vaccenic acid.

More preferred exemplary combinations include malic acid, hydroxysuccinic acid, hydroxyglutaric acid or tartaric acid with palmitoleic acid, sapienic acid, oleic acid, elaidic acid or vaccenic acid, especially palmitoleic acid or oleic acid.

Another more preferred exemplary combination includes malic, tartaric or succinic acid with palmitoleic or oleic acid.

An especially more preferred exemplary combination includes malic acid with palmitoleic or oleic acid.

An especially most preferred exemplary combination is malic acid with oleic acid.

Additional combinations may be summarized as abbreviated lists in which the base line dicarboxylic acids, preferred dicarboxylic acids and more preferred dicarboxylic acids (acronyms DA, PDA, MPDA) and base line fatty monocarboxylic acids, preferred fatty monocarboxylic acids and more preferred fatty monocarboxylic acids (acronyms FA, PFA, MPFA) are categorized as described above. These categories are presented as the following tabular lists.

### Diacid (DA) Embodiments

1. Saturated linear C2 - C13 diacid = DA1
2. Saturated linear C3 - C13 diacid with 1 or 2 hydroxyls = DA2

### Preferred Diacid (PDA) Embodiments

1. C3-C10 Diacid saturated = PDA1
2. C3-C10 Diacid saturated with 1 hydroxyl = PDA2
3. C3-C10 Diacid saturated with 1 or more hydroxyls = PDA3

### More Preferred Diacid (MPDA) Embodiments

1. C3-C6 Diacid saturated with one hydroxyl = MPDA1
2. C4-C6 Diacid saturated with 2 or 3 hydroxyls = MPDA2

### Fatty Acid (FA) Emdodiments

1. Saturated C10 - C30 Fatty acid = FA1
2. Unsaturated C10 - C30 Fatty acid with 1 unsaturation = FA2
3. Unsaturated C10 - C30 Fatty acid with 2 unsaturations = FA3

### Preferred Fatty Acid (PFA) Embodiments

1. Saturated C14 - C22 Fatty acid = PFA1
2. Unsaturated C14 - C22 Fatty acid with 1 unsaturation = PFA2

### More Preferred Fatty Acid (MPFA) Embodiments

1. Unsaturated C16 - C18 Fatty acid with 1 unsaturation = MPFA1
The combinations of the foregoing categories of dicarboxylic acid and fatty monocarboxylic acid include:
DA +FA Combinations
   DA1 + FA1; DA1 + FA2; DA1+ FA3
   DA2 + FA1; DA2 + FA2; DA2 + FA3
PDA + FA Combinations
   PDA1 + FA1; PDA1 + FA2; PDA1+ FA3
   PDA2 + FA1; PDA2 + FA2; PDA2 + FA3
   PDA3 + FA1; PDA3 + FA2; PDA3 + FA3
MPDA + FA Combinations
   MPDA1 + FA1; MPDA1 + FA2; MPDA1+ FA3
   MPDA2 + FA1; MPDA2 + FA2; MPDA2 + FA3
DA +PFA Combinations
   DA1 + PFA1; DA1 + PFA2; DA1+PFA3
   DA2 + PFA1; DA2 + PFA2; DA2 + PFA3
PDA + PFA Combinations
   PDA1 + PFA1; PDA1 + PFA2
   PDA2 + PFA1; PDA2 + PFA2
   PDA3 + PFA1; PDA3 + PFA2
MPDA + PFA Combinations
   MPDA1 + PFA1; MPDA1 + PFA2
   MPDA2 + PFA1; MPDA2 + PFA2
DA +MPFA Combinations
   DA1 + MPFA1
   DA2 + MPFA1
PDA + MPFA Combinations
   PDA1 + MPFA1
   PDA2 + MPFA1
   PDA3 + MPFA1
MPDA + MPFA Combinations
   MPDA1 + MPFA1
   MPDA2 + MPFA1

The concentration ranges of the dicarboxylic acid and the fatty monocarboxylic acid follow a general relationship in which the weight percentage relative to the total weight of the composition may usually but not always be larger for the dicarboxylic acid than for the fatty monocarboxylic acid. This weight percentage is calculated based on the total weight combination of dicarboxylic acid and the fatty monocarboxylic acid without any other component. The weight percent ratio of dicarboxylic acid to fatty monocarboxylic acid ranges from about 50:1 to about 1:10, preferably about 30:1 to about 1:7.5, more preferably about 30:1 to about 1:5, especially more preferably about 30:1 to about 1:3 or to about 1:75, with exemplary wt ratios being up to 30:1, 20:1, 10:1, 6:1, 3:1, 1: 1. 1: 1.67, 1:2, 1:3, 1:5 and 1:10.

Each of these components also has an individual concentration range in weight percentage relative to the composition total weight of 100% wherein the composition total weight includes all other components such as at least a medium and the secondary component if present.

The individual concentration ranges for the primary component constituents follows the above-described concentration relationship. The dicarboxylic acid may be present at about 0.01 wt% to about 35 wt%, preferably about 0.1 wt% to 25 wt%, more preferably from about 0.1 wt% to about 20 wt%, more preferably from about 0.1 wt% to about 15 wt%. Exemplary concentrations of the dicarboxylic acid include 0.1, 0.5. 1.0, 1.5, 2, 2.5, 3, 3.5, 4, 4.5, 5, 5.5, 6, 6.5, 7, 7.5, 8, 8.5, 9, 9.5 and 10 wt%'s and all digital integrals between these numbers.

The fatty monocarboxylic acid may be present at about 0.01 wt% to about 35 wt%, preferably about 0.015 wt% to about 25 wt%. more preferably about 0.015 wt% to about 20 wt%, especially more preferably about 0.015 wt% to about 15 wt%. Exemplary concentrations of the fatty carboxylic acid include 0.01, 0.015, 0.03, 0.1, 0.2, 0.5. 0.75, 1.0, 1.5, 2, 2.5, 3, 3.5, 4, 4.5, 5, 5.5, 6, 6.5, 7, 7.5, 8, 8.5, 9, 9.5 and 10 wt%'s and all digital integrals between these numbers.

The carboxylic acid embodiments of the primary component may be present in the hair treatment composition as acids and/or as salts of the carboxylic acid groups wherein the counter ion in the salt may be sodium, potassium and/or ammonium. The pH of the hair treatment composition may be adjusted with acid or base and/or with buffer as described below. The pH adjustment may but not necessarily convert at least in part the carboxylic acid groups of the carboxylic acid embodiments to salts and/or buffered salts.

Anagenic hair can be damaged by wear and degradation caused by perming, redox treatment, shampooing, cleansing and cleaning with anionic surfactants, brushing, combing and/or rubbing such as with towels, hats, caps and the like. These activities performed on anagenic hair strands cause the strands to be thinned, broken, fragmented, splintered and otherwise damaged. This damage is believed to result from lift of the cellular scales on the surface of the cuticles of the hair strands and removal of lubricating substances from the scales. The lift and de-lubrication enable breakage, dislodgement, fragmentation, and disruption of the inter-scale connections of fatty acids, phospholipids and disulfide bonds. Water, soap, shampoo, anionic surfactants and similar solubilization agents are able to permeate the cuticle as a result of the lifted individual scales of the cuticle. This permeation enables dilution, solubilization and dispersion of the lipids, fats, fatty acids and sebum which form at least in part micro connections between the lifted scales. The result is a weakening of the hair strand cuticle leading to breakage, fragmentation and splintering of the strand.

The above-described embodiments of the hair treatment composition according to the invention replace and reincorporate the inter-scale connections of hair strands, coat hair strand scale surfaces and close inter-scale spaces resulting from lift. The result is believed to strengthen the hair strands and render them less susceptible to lift. The strengthening can be measured by the ease of combability of such treated hair strands.

### The Optional Secondary Component

The primary component of the hair treatment composition may be combined with an optional secondary component which comprises any one or more of the lipophilic hydrocarbon and fatty acid ester constituents. The additional secondary constituents include but are not limited to a fatty alcohol constituent, a cationic surfactant constituent, a silicone constituent, a cationic polymer constituent, a nonionic surfactant constituent, a preformed polymer constituent, an emulsifier constituent, a thickener constituent, a coloring constituent, and one or more of other additive constituents.

Hair treatment composition comprising the primary component and the secondary component may have any one or more of the constituents of the secondary component present at concentrations ranging from about 0.0 wt% (i.e., not present as indicated by optional) to about 0.01 wt% to about 40.0 wt%, preferably up to 35 wt%, more preferably up to 30 wt%, especially more preferably up to 20 wt%, even more especially preferably up to 15 wt%, and all 1 wt% steps from 0.01 wt% to these maximum values are included. These concentrations are relative to the total weight of the entire composition including at least the medium, the primary component and secondary component. The weight percentage ratio of the primary component (sum of dicarboxylic acid and fatty monoacid) to the secondary component (sum of all secondary components) may range from about 20:1 to about 1:20, preferably about 10:1 to about 1:10, more preferably about 5:1 to about 1:5, especially about 4:1 to about 1:4 and all ratios between these ranges.

### Lipid Constituent

Embodiments of the lipid constituent of the second component are directed to one or more of a fatty acid ester and/or a long chain "fatty" saturated and/or unsaturated hydrocarbon, preferably having multiple pendant methyl groups or a hydrocarbon mimic such as a silicone oil or natural oil.

For embodiments of the lipid constituent comprising the fatty acid ester version, the acid is the saturated or unsaturated fatty acid as described above for the primary component including the preferred and more preferred versions thereof and the esterifying alcohol may be a mono, di or tri hydroxyl alkane of 2 to 10 carbons. The hydroxyl variation of the alcohol provides at least five versions of the ester depending upon how many hydroxyls of the esterifying alcohol are converted to ester groups. These are an alkyl fatty carboxyl ester (using a monoalcohol) , an alkyl di-fatty carboxyl ester (using both hydroxyls of a diol), a hydroxyalkyl fatty carboxyl ester (using one hydroxyl of a diol), a di-hydroxyalkyl fatty carboxyl ester (using one hydroxyl of a triol), a hydroxyalkyl di-fatty carboxyl ester (using two hydroxyls of a triol) and an alkyl tri-fatty carboxyl ester (using three hydroxyls of a triol). Preferred among these versions of the fatty acid ester are the hydroxyalkyl fatty carboxyl ester (using one hydroxyl of a diol), a di-hydroxyalkyl fatty carboxyl ester (using one hydroxyl of a triol) and an alkyl di-fatty carboxyl ester (using both hydroxyls of a diol). Preferred diols include a C2-C6 diol, more preferably a C2-C4 diol. Preferred triols include a C3-C6 triol, more preferably glycerin.

Basic, preferred and more preferred embodiments of the fatty acid ester constituent of the secondary component may be summarized in the following tables.

### Base Fatty Acid Ester (FAE) Embodiments

1. Saturated C10 - C30 Fatty acid with C1-C10 mono alcohol = FAE1
2. Unsaturated C10 - C30 Fatty acid with 1 unsaturation with C1-C10 mono alcohol = FAE2
3. Unsaturated C10 - C30 Fatty acid with 2 unsaturations with C1-C10 mono alcohol = FAE3
4. Saturated C10 - C30 Fatty acid with C2-C10 diol alcohol, 1 ester = FAE4
5. Unsaturated C10 - C30 Fatty acid with 1 unsaturation with C2-C10 diol alcohol, 1 ester = FAE5
6. Unsaturated C10 - C30 Fatty acid with 2 unsaturations with C2-C10 diol alcohol, 1 ester = FAE6
7. Saturated C10 - C30 Fatty acid with C2-C10 diol alcohol, 2 ester groups = FAE7
8. Unsaturated C10 - C30 Fatty acid with 1 unsaturation with C2-C10 diol alcohol, 2 ester groups = FAE8
9. Unsaturated C10 - C30 Fatty acid with 2 unsaturations with C2-C10 diol alcohol, 2 ester groups = FAE9
10. Saturated C10 - C30 Fatty acid with C3-C10 triol alcohol, 1 ester = FAE10
11. Unsaturated C10 - C30 Fatty acid with 1 unsaturation with C3-C10 triol alcohol, 1 ester = FAE11
12. Unsaturated C10 - C30 Fatty acid with 2 unsaturations with C3-C10 triol alcohol, 1 ester = FAE12
13. Saturated C10 - C30 Fatty acid with C3-C10 triol alcohol, 2 ester groups = FAE13
14. Unsaturated C10 - C30 Fatty acid with 1 unsaturation with C3-C10 triol alcohol, 2 ester groups = FAE14
15. Unsaturated C10 - C30 Fatty acid with 2 unsaturations with C3-C10 triol alcohol, 2 ester groups = FAE15
16. Saturated C10 - C30 Fatty acid with C3-C10 triol alcohol, 3 ester groups = FAE16
17. Unsaturated C10 - C30 Fatty acid with 1 unsaturation with C3-C10 triol alcohol, 3 ester groups = FAE17
18. Unsaturated C10 - C30 Fatty acid with 2 unsaturations with C3-C10 triol alcohol, 3 ester groups = FAE18

### Preferred Fatty Acid Ester (PFAE) Embodiments

1. Unsaturated C14 - C22 Fatty acid with 1 unsaturation with C1-C6 mono alcohol = PFAE1
2. Unsaturated C14 - C22 Fatty acid with 1 unsaturation with C2-C6 diol alcohol, 1 ester = PFAE2
3. Unsaturated C14 - C22 Fatty acid with 1 unsaturation with C2-C6 diol alcohol, 2 ester groups = PFAE3
4. Unsaturated C14 - C22 Fatty acid with 1 unsaturation with C3-C10 triol alcohol, 2 ester groups = PFAE4
5. Unsaturated C14 - C22 Fatty with 1 unsaturation with C3-C6 triol alcohol, 3 ester groups = PFAE5

### More Preferred Fatty Acid Ester (MPFAE) Embodiments

1. Unsaturated C16 - C18 Fatty acid with 1 unsaturation with C2-C6 mono alcohol = MPFAE1
2. Unsaturated C16 - C18 Fatty acid with 1 unsaturation with C2-C4 diol alcohol, 2 ester groups = MPFAE2
3. Unsaturated C16 - C18 Fatty acid with 1 unsaturation with C3-C4 triol alcohol, 3 ester groups = MPFAE3

For embodiments of the lipid constituent of the secondary component comprising the hydrocarbon or hydrocarbon mimic, the hydrocarbon may be saturated or unsaturated and linear or branched comprising a hydrocarbon chain ranging in carbon count from 10 to 40 carbons, preferably 20 to 40 carbons. Preferably, the hydrocarbon is branched with methyl groups pendant to the hydrocarbon chain. Preferably the unsaturation ranges for two to six sites of unsaturation.

Exemplary hydrocarbons include liquid petroleum comprising a mixture of linear and/or branched C10 - C30 hydrocarbon chains. Additional exemplary hydrocarbons include mono-terpenes (C10) di-terpenes (C20), tri-terpenes (C30) sesqui versions thereof (C15, C25) and exemplary terpenes such as 2,6-dimethyl octane, 2,6-dimethyl octene, 2-methyl-6-ethyl heptane, 2-methyl-6-ethyl heptene, α- and/or β- farnesane, isofarnesane, neofarnesane, farnesene, isofarnesene, neofarnesene, pristane, isopristane, neopristane, pristene, isopristene, neopristene, phytane isophytane, neophytane, phytene, isophytene, neophytene, squalane, isosqualane, neosqualane, squalene, isosqualene, neosqualene or any combination thereof as well as liquid hydrogenated polyisoprene, isoparaffin, mineral oil and liquid olefin polymer wherein the hydrocarbon polymer is liquid at standard pressure and temperature.

The preferred hydrocarbon may be squalane or a pendant multi-methyl substituted C24-C30 saturated hydrocarbon chain or mixtures thereof.

Exemplary hydrocarbon mimics include silicone oil, natural oils such as olive oil, soybean oil, corn oil, coconut oil, rape seed oil, flax seed oil, argan oil, jojoba oil and passion fruit oil.

Additional alternative hydrocarbon embodiments include vegetable oils such as but not limited to soybean oil, corn oil, palm oil, rape seed oil, peanut oil, coconut oil, olive oil, almond oil, cashew oil, canola oil and others; all purified to remove fatty acid residues.

Basic, preferred and more preferred embodiments of the hydrocarbon constituent of the secondary component may be summarized in the following lists.

### Hydrocarbon (HC Embodiments, each branched with from 3 to 6 branch methyl groups)

1. C10 - C40 hydrocarbon all saturated
2. C10 - C40 hydrocarbon 1 unsaturation
3. C15 - C40 hydrocarbon 2 unsaturations
4. C20 - C40 hydrocarbon 3 unsaturations
5. C20 - C40 hydrocarbon 6 unsaturations

### Preferred Hydrocarbon (PHC) Embodiments

1. C15 - C30 hydrocarbon all saturated
2. C15 - C30 hydrocarbon 3-6 unsaturations

### More Preferred Hydrocarbon (MPHC) Embodiments

1. Squalene
2. Squalane

Embodiments of the lipid constituent of the second component may comprise at least the fatty ester alone or the hydrocarbon alone, preferably the fatty ester alone. The lipid constituent may alternatively comprise the combination of the hydrocarbon and the fatty ester.

A more preferred embodiment of the optional secondary component for inclusion with the primary component as the lipid constituent comprises the fatty acid ester. The preferred fatty acid ester comprises the ester product formed from the alcohol moiety comprising the di or tri hydroxyl alkane and the preferred fatty monocarboxylic acid embodiment of the organic acid component.

When the lipid constituent of the optional secondary component is added to the hair treatment composition comprising any of the stand-alone embodiments, the lipid component most preferably may be a mono fatty acid ester formed with a triol and liquid petrolatum or a PHC or MPHC hydrocarbon .

### Additional Constituents of the Optional Second Component

Additional optional constituents of the optional second component for embodiments of the hair treatment composition depend at least in part upon the purpose and function of the hair treatment composition. If the hair treatment composition is to be a part of a general purpose conditioner or a rinse off or leave on conditioner or a mask, a cationic surfactant and/or a cationic polymer and/or a nonionic surfactant may be included. A fatty alcohol, a fatty alcohol ester, a moisturizer, a humectant, a thickener, an emulsifier as well as other additives for delivering advantageous qualities such as but not limited to coloration touch-up may be included the hair treatment composition especially when used as the leave-on and mask embodiments. A nonionic surfactant may be included to enable development of at least substantially homogeneous mixtures and uniform distributions on the hair of the embodiments of the hair treatment composition. In addition, when additional components are lipophilic in nature, they may be included as part of the lipophilic factor of the optional secondary component and their concentrations are included as a part of the concentration ranges for the optional secondary component.

### Optional Cationic Surfactant

An optional additive of the secondary component of the composition of the present invention may comprise a cationic surfactant. The cationic surfactant may comprise one cationic surfactant or a mixture of two or more cationic surfactants. When present, the cationic surfactant may be included in the composition at a level by weight of from about 0.1% to about 10%, preferably from about 0.5% to about 8%, more preferably from about 0.8% to about 5%, still more preferably from about 1.0% to about 4% relative to the total weight of the composition.

The cationic surfactant constituent of at least some embodiments of the hair treatment composition of the invention may comprise a quaternary ammonium compound, a tertiary amine compound, an amido ammonium and/or tertiary amine compound, a mono, di and tri polysaccharide with quaternary ammonium or tertiary amine groups, or any mixture thereof.

Non-limiting classes of these cationic surfactants include a mono or di fatty alkyl or alkenyl quaternary ammonium and tertiary amine compound, a mono or di polyol quaternary ammonium and tertiary amine compound, a mono or di fatty alkyl or alkenyl benzyl quaternary ammonium and tertiary amine compound, a fatty alkyl or alkenyl aryl, polyol quaternary ammonium and tertiary amine compound, a polyol mono, di or tri saccharide alkyl quaternary ammonium and tertiary amine compound, a tertiary or quaternized amino alkyl fatty alkyl or alkenyl ester or amide compound, a phenoxy-alkyl fatty alkyl or alkenyl quaternary ammonium and tertiary amine compound, a hydrolyzed starch with terminally quaternized ammonium and tertiary amine, a multi-polyol cellulose with terminal quaternized ammonium and tertiary amine, sucrose, lactose, a mono and disaccharide, arabic, ghatti, guaicum, guar, karaya, locust bean and a xanthan gum derivatized with terminal quaternized ammonium and tertiary amine groups , and similar hydrophobic tail with cationic head organic compounds. The classes of cationic surfactants with tertiary amine groups may be protonated by the media or by organic acid to form cationic groups.

Examples of the cationic surfactant may be selected from and/or include cetrimonium chloride, steartrimonium chloride, behentrimonium chloride, behentrimonium methosulfate, behenamidopropyltrimonium methosulfate, stearamidopropyltrimonium chloride, arachidtrimonium chloride, distearyldimonium chloride, dicetyldimonium chloride, tricetylmonium chloride, oleamidopropyl dimethylamine, linoleamidopropyl dimethylamine isostearamidopropyl dimethylamine, oleyl hydroxyethyl imidazoline, stearamidopropyldimethylamine, behenamidopropyldimethylamine, behenamidopropyldiethylamine, behenamidoethyldiethyl-amine, behenamidoethyldimethylamine, arachidamidopropyldimethylamine, arachidamido-propyidiethylamine, arachidamidoethyidiethylamine, arachidamidoethyidimethylamine, benzalkonium chloride, benzodidecinium bromide, cetalkonium chloride, dicedyldimenthlammonium chloride dimethyldioctadecylamminum chloride, dioleoyl-3-trimethyl ammonium propane, lauryl methyl gluceth-10 hydroxylpropyl dimonium chloride N-oleyl-1,3-propane diamine, stearalkonium chloride and mixtures thereof. Additional cationic surfactants include cetyl trimethyl ammonium chloride available, for example, with trade name CA-2350 from Nikko Chemicals and CTAC 30KC available from KCl, stearyl trimethyl ammonium chloride with trade name Arquad 18/50 available from Akzo Nobel, hydrogenated tallow alkyl trimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyl colamino formyl methyl) pyridinium chloride; hydrophilically substituted cationic surfactants having the following INCI designations: quaternium-16, quaternium-26, quaternium-27, quaternium-30, quaternium-33, quaternium-43, quaternium-52, quaternium-53, quaternium-56, quaternium-60, quaternium-61, quaternium-62, quaternium-70, quaternium-71, quaternium-72, quaternium-75, quaternium-76 hydrolyzed collagen, quaternium-77, quaternium-78, quaternium-79 hydrolyzed collagen, quaternium-79 hydrolyzed keratin, quaternium-79 hydrolyzed milk protein, quaternium-79 hydrolyzed silk, quaternium-79 hydrolyzed soy protein, and quaternium-79 hydrolyzed wheat protein, quaternium-80, quaternium-81, quaternium-82, quaternium-83, quaternium-84, and any combination thereof.

In particular, a preferred cationic surfactant for inclusion with the hair treatment composition of the invention comprises a "Quat" which is a quaternized ammonium salt having at least one linear, long alkyl chain having from about 12 to about 30 carbon atoms, preferably from about 16 to about 24 carbon atoms, more preferably about C18 to about C22. Typically the "Quat" may comprise one or two long alkyl chains and preferably may comprise one long alkyl chain. The remaining groups attached to nitrogen of such a "Quat" are independently selected from an alkyl group of from 1 to about 4 carbon atoms or a hydroxyalkyl, aryl or alkylaryl group having up to about 14 carbon atoms. Exemplary Quats are those embodiments described above.

### Optional Silicone Conditioning Agent

Another optional additive for the secondary component of the hair treatment composition of the present invention may also comprise at least one silicone conditioning agent. Examples of the silicone include dimethicone, dimethiconol, a cyclic silicone, and a modified silicone with various functional groups such as amino groups, quaternary ammonium salt groups, and an amine silicone such as Amodimethicone. Such silicones may be soluble or insoluble in the aqueous (or non-aqueous) medium. In the case of insoluble liquid silicones, the polymer can be in an emulsified form with droplet size of about 10 nm to about 30 micrometers.

When present, the silicone may be included in the composition at a level by weight of from about 0.1% to about 10%, preferably from about 0.5% to about 8%, more preferably from about 0.8% to about 5%, still more preferably from about 1.0% to about 4% relative to the total weight of the composition.

### Optional Fatty Alcohol

A fatty alcohol may be included as an additional, optional constituent of the second component in embodiments of the stand-alone conditioning and mask embodiments of the hair treatment compositions of the invention. The fatty alcohol provides emulsifying, occlusive, moisturizing, thickening and humectant properties to the hair treatment composition. Included are at least one fatty alcohol and mixtures thereof having a linear or branched carbon chain of from about 10 to about 30 carbons. The fatty alcohol may be saturated or unsaturated. Exemplary fatty alcohols include lauryl, tridecyl, myristyl pentadecyl, palmitoleyl (unsaturated) hexadecyl, stearyl, oleyl (unsaturated), nonadecyl, arachidyl, heneicosyl, benenyl, erucyl, lignoceryl, ceryl, heptacosanyl, montanyl, nonacosanyl, myricyl, dotriacontanyl and geddyl alcohol. Preferred are lauryl alcohol, myristyl alcohol, cetyl alcohol, stearyl alcohol, behenyl alcohol, oleyl alcohol, arachidyl alcohol and mixtures thereof such as cetearyl alcohol (mixture of cetyl and stearyl alcohols).

When present, the fatty alcohol may be included in the composition at a level by weight of from about 0.1% to about 15%, preferably from about 0.5% to about 10%, more preferably from about 0.8% to about 10%, still more preferably from about 1.0% to about 8% relative to the total weight of the composition.

### Optional Fatty Alcohol Ester

The optional secondary component may also include as an additional optional constituent a fatty alcohol ester. The fatty alcohol ester is composed of an esterified acid which is a monocarboxylic acid of 3 to 10 carbons such as acetic, propionic, butyric, valeric, caproic, enanthic, caprylic pelargonic, capric acid or the linear dicarboxylic acid of 3 to 10 carbons without hydroxyl as described above for the primary component. The fatty alcohol is the fatty alcohol of 10 to 30 carbons as described above. The dicarboxylic acid for the fatty alcohol ester version includes the above described preferred and more preferred versions thereof also. The fatty alcohol may be saturated or unsaturated including such fatty alcohols as lauryl, tridecyl, myristyl, penatadecyl cetyl, palmitoleyl, heptadecyl, stearyl, oleyl, nonadecyl, arachidyl, heneicosyl, behenyl, erucyl, lignoceryl, ceryl, heptacosanyl, montanyl, nonacosanyl and myricyl alcohol with the unsaturated fatty alcohols such as palmitoleyl, oleyl, heneicosyl and erucyl alcohol being preferred. The carboxyl variation of the acid provides at least three versions of the ester. These are a monocarboxylic acid esterified with a fatty alcohol, a dicarboxylic acid esterified once with a fatty alcohol and a dicarboxylic acid esterified twice with a fatty alcohol. Additionally, the lipid character of the single esterification of the dicarboxylic acid may be enhanced by esterifying the remaining carboxyl group with a monoalcohol of 1 to 6 carbons.

### Optional Cationic Polymer

At least one cationic polymer may also be included as an optional additional constituent of the secondary component. A cationic polymer useful herein may have an average molecular weight of at least about 5,000, typically from about 10,000 to about 10 million, preferably from about 100,000 to about 2 million. The cationic polymer differs from the cationic surfactant because of its polymeric character and the number of nitrogen groups in the molecule. The cationic polymer has repeating units of olefinic, ester, amide, urea and/or urethane monomer residues or carbohydrate (polysaccharide or cellulose) residues while the cationic surfactant has non-polymeric aliphatic groups. The cationic polymer contains multiple nitrogen groups as amines or quaternary ammonium groups or has multiple phosphonium groups while the cationic surfactant contains a single nitrogen group as an amine or quaternary ammonium group.

Suitable cationic polymers include, for example, copolymers of vinyl monomers having cationic amine or quaternary ammonium functionalities with optional water soluble spacer monomers or non-polar olefinic spacer monomers. The water soluble spacer monomers include such polar monomers as acrylamide, methacrylamide, alkyl and dialkyl acrylamides, alkyl and dialkyl methacrylamides, alkyl acrylate, alkyl methacrylate, vinyl caprolactone, and vinyl pyrrolidone. Other suitable spacer monomers include vinyl ester, vinyl alcohol (made by hydrolysis of polyvinyl acetate), maleic anhydride, propylene glycol, and ethylene glycol. The non-polar olefinic spacer monomers include ethylene, propylene, butene, isoprene, styrene and similar olefin monomers. Other suitable cationic polymers useful herein include, for example, a cationic celluloss, a cationic starch, and a cationic guar gum wherein the cationic groups are ammonium or phosphonium groups.

Exemplary cationic polymers include but are not limited to polyquaternium-1, polyquaternium-2, polyquaternium-4, polyquaternium-5, polyquaternium-6, polyquaternium-7, polyquaternium-8, polyquaternium-9, polyquaternium-10, polyquaternium-11, polyquaternium-12, polyquaternium-13, polyquaternium-14, polyquaternium-15, polyquaternium-16, polyquaternium-17, polyquaternium-18, polyquaternium-19, polyquaternium-20, polyquaternium-22, polyquaternium-24, polyquaternium-27, polyquaternium-28, polyquaternium-29, polyquaternium-30, polyquaternium-31, polyquaternium-32, polyquaternium-33, polyquaternium-34, polyquaternium-35, polyquaternium-36, polyquaternium-37, polyquaternium-38, polyquaternium-39, polyquaternium-43, polyquaternium-44, polyquaternium-45, polyquaternium-46, polyquaternium-47, polyquaternium-48, polyquaternium-49, polyquaternium-50, polyquaternium-52, polyquaternium-53, polyquaternium-54, polyquaternium-55, polyquaternium-56, polyquaternium-57, polyquaternium-58, polyquaternium-59, polyquaternium-60, polyquaternium-63, polyquaternium-64, polyquaternium-65, polyquaternium-66, polyquaternium-67, polyquaternium-70, polyquaternium-73, polyquaternium-74, polyquaternium-75, polyquaternium-76, polyquaternium-85, polyquaternium-86, polybeta-alanine, polyepsilon-lysine, polylysine, PEG-8/SMDI copolymer, PPG-12/SMDI copolymer, PPG-51/SMDI copolymer, PPG-7/succinic acid copolymer, IPDI/PEG-15 cocamine copolymer, IPDI/PEG-15 cocamine copolymer dimer dilinoleate, IPDI/PEG-15 soyamine copolymer, IPDI/PEG-15 soyamine oxide copolymer, IPDI/PEG-15 soyethonium ethosulfate copolymer, polyquaternium-4/hydroxypropyl starch copolymer, cassia hydroxypropyltrimonium chloride, chitosan hydroxypropyltrimonium chloride, dextran hydroxypropyltrimonium chloride, galactoarabinan hydroxypropyltrimonium chloride, ginseng hydroxypropyltrimonium chloride, guar hydroxypropyltrimonium chloride, hydroxypropyl guar hydroxypropyltrimonium chloride, locust bean hydroxypropyltrimonium chloride, starch hydroxypropyltrimonium chloride, hydroxypropyltrimonium hydrolyzed corn starch, hydroxypropyl oxidized starch PG-trimonium chloride, tamarindus indica hydroxypropyltrimonium chloride, polyacrylamidopropylltrimonium chloride, polymethacrylamidopropylltrimonium chloride, polyacrylamidopropylltrimonium methosulfate, propyltrimoniumchloride methacrylamide/dimethylacrylamide copolymer, acrylamide/ethalkonium chloride acrylate copolymer, acrylamide/ethyltrimonium chloride acrylate/ethalkonium chloride acrylate copolymer, acrylates/carbamate copolymer, adipic acid/methyl DEA crosspolymer, diethylene glycol/DMAP acrylamide/PEG-180/HDI copolymer, dihydroxyethyl tallowamine/IPDI copolymer, dimethylamine/ethylenediamine/epichlorohydrin copolymer, HEMA glucoside/ethylmethacrylate trimonium chloride copolymer, hydrolyzed wheat protein/PEG-20 acetate copolymer, hydrolyzed wheat protein/PVP crosspolymer, ethyltrimonium chloride methacrylate/hydroxyethylacrylamide copolymer.

The cationic polymer can be included in the hair conditioning composition of the present invention at a level of from about 0.001 wt% to about 10 wt%, preferably from about 0.005 wt% to about 5 wt%, more preferably about 0.01 wt% to about 5 wt% most preferably from about 0.1 wt% to about 5 wt% relative to the total weight of the composition.

### Optional Emulsifiers, Non-ionics and Suspending Agents

Optional additional constituents for optional inclusion in the second component of the hair treatment composition of the present invention may also comprise emulsifiers, non-ionic surfactants and suspending agents for maintaining substantially homogeneous dispersions and suspending optional micro-particulates in the hair treatment composition. Useful crystalline suspending agents include those categorized as acyl derivatives, long chain amine oxides, and mixtures thereof. These suspending agents are described in U.S. Pat. No. 4,741,855. These suspending agents include ethylene glycol esters of fatty acids in one aspect having from about 16 to about 22 carbon atoms. In one aspect, useful suspending agents include ethylene glycol stearates, both mono and distearate, but in one aspect, the distearate containing less than about 7% of the mono stearate. Other suitable suspending agents include alkanol amides of fatty acids, having from about 16 to about 22 carbon atoms, or even about 16 to 18 carbon atoms, examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); long chain esters of long chain alkanol amides (e.g., stearamide diethanolamide distearate, stearamide monoethanolamide stearate); and glyceryl esters (e.g., glyceryl distearate, trihydroxystearin, tribehenin), Long chain acyl derivatives, ethylene glycol esters of long chain carboxylic acids, long chain amine oxides, and alkanol amides of long chain carboxylic acids in addition to the materials listed above may be used as suspending agents. Other long chain acyl derivatives suitable for use as suspending agents include N,N-dihydrocarbyl amido benzoic acid and soluble salts thereof (e.g., Na, K), particularly N,N-di(hydrogenated) C16, C18 and tallow amido benzoic acid species of this family, which are commercially available from Stepan Company (Northfield, Ill., USA).

Examples of suitable long chain amine oxides for use as suspending agents include alkyl dimethyl amine oxides, e.g., stearyl dimethyl amine oxide. Other suitable suspending agents include primary amines having a fatty alkyl moiety having at least about 16 carbon atoms, examples of which include palmitamine or stearamine, and secondary amines having two fatty alkyl moieties each having at least about 12 carbon atoms, examples of which include dipalmitoylamine or di(hydrogenated tallow)amine Still other suitable suspending agents include di(hydrogenated tallow)phthalic acid amide, and crosslinked maleic anhydride-methyl vinyl ether copolymer.

### Optional Non-ionic Surfactant

A non-ionic surfactant may optionally be included as an additive of the second component for the stand-alone conditioner and mask embodiments of the hair treatment composition according to the invention. The addition of the non-ionic surfactant is believed to provide an additional homogenizing component to the hair treatment composition so that highly hydrophobic optional constituents such as hydrocarbons, alumino-silicate clays such as bentonite, hectorite and the like and fatty alcohols can be substantially to essentially to completely homogeneously formulated into the aqueous or aqueous organic medium of the hair treatment composition according to the invention.

Non-ionic surfactants useful for these purposes in the hair treatment composition comprise the classes of fatty acid esters of polyethylene oxide (PEG) and poly propylene oxide, and fatty alcohol esters and ethers of fatty alcohol and /PEG and/or PPG as esterifying alcohols of C3-C6 saturated dicarboxylic acid. The fatty alcohol ethers useful herein include alkoxylated fatty alcohols, alkyl ethers of alkoxylated fatty alcohols and mixtures thereof. Nonlimiting examples of fatty alcohol derivatives include materials such as methyl stearyl ether; the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through steareth-10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, (e.g. a mixture of fatty alcohols containing predominantly cetyl and stearyl alcohol, wherein the numeric designation indicates the number of ethylene glycol moieties present); C1-C30 alkyl ethers of the ceteth, steareth, and ceteareth compounds just described; polyoxyethylene ethers of behenyl alcohol; polyoxyethylene cetyl ether stearate, polyoxyethylene stearyl ether stearate, polyoxyethylene lauryl ether stearate, polyoxyethylene monostearate, polyoxyethylene distearate, propyleneglycol monostearate, sorbitan stearate, polyglyceryl stearate and mixtures thereof.

Additional nonionic surfactants include an acyl-amino deoxy sugar, an acyl-amino sugar alcohol and an alkyl glycoside. The acyl amino deoxy sugar and acyl amino sugar alcohol have the formula R¹-CO-NX-R² wherein R¹-CO is a linear or branched C6 - C30 fatty acyl group with R¹ being a linear or branched C5-C29 alkyl group. R² is hydrogen or C1-C6 alkyl. The moiety - NX is amino deoxy sugar or amino sugar alcohol. The amino deoxy sugar group has the open form formula 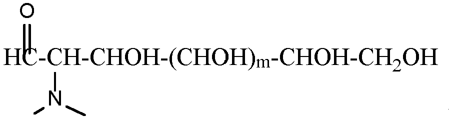 with m as zero or 1. The amino sugar alcohol group has the formula =N-CH₂-CHOH-CHOH-(CHOH)ₘ-CHOH-CH₂OH with m being zero or 1. Examples of amino sugars and amino sugar alcohols include the -NH₂ derivatives of deoxy pentose, deoxy hexose,di-deoxyhexose and the corresponding alcohols. The corresponding deoxy sugar core examples include but are not limited to deoxyglucose, deoxymannose, deoxyidose, deoxytalose, deoxygalactose, deoxyribose deoxyxylose, doxyarabinose, deoxylyxose; and sugar alcohol core examples include but are not limited to xylitol, ribitol, mannitol, sorbitol, galactitol, fucitol, iditol, inositol, volemitol, isomalt, maltitol, lactitol. Preferable amino deoxy sugars and amino sugar alcohols include glucosamine, amino deoxy mannose, amino deoxy galactose, aminosorbitol, aminomannitol and amino deoxyribose. More preferable is glucosamine. The preferable acyl-amino deoxysugars include lauroyl/myristyl methyl glucamide, capryloyl/caproyl methyl glucamide, capryloyl/caproyl glucamide.

The alkyl glycoside class of non-ionic surfactant serving as a component of the hair treatment composition according to the invention comprises C8-C30 linear and branched alkyl glycosides. The glycoside cores of this class includes but is not limited to sugar moieties such as glucosides, fructosides, glucuronides, mannosides, galactosides, ribosides as well as polyglycosides such as polyglucoside, polyfructoside, polymannoside, polygalactoside and polyglucuronic acid. The alkyl group is derived from a C8-C30 fatty alcohol and forms a glycosidic bond with the anomeric carbon (the carbonyl carbon) of the sugar through formation of a ketal group from the carbonyl group. Exemplary glycosides include but are not limited to lauryl glucoside, decyl glucoside, coco glucoside, octyl glucoside, capryl glucoside, caprylyl/capryl glucoside, decyl polyglucoside, coco polyglucoside, lauryl polyglucoside and mixtures thereof.

The concentration of non-ionic surfactant when included in the hair treatment composition according to the invention ranges from about 0.2 wt% to about 10 wt%, preferably about 0.2 wt% to about 5 wt%, more preferably about 0.2 wt% to about 2 wt% relative to the total weight of the composition.

### Optional Betaines and Amidobetaines

Another optional additive for the second component may include a betaine and/or an amidobetaine to provide humectant, surfactant, antistatic, conditioning, foam boosting and thickening properties. Useful betaines include a trialkylammonium alkyl carboxylate and a trialkylammonium phenyl carboxylate in which the nitrogen is quaternized with three C1-C3 alkyl groups and the C1-C4 alkyl or phenyl linker with the carboxylate. The zwitterionic character of the betaines enables polar properties while a larger sized linker delivers emulsifying properties to compositions containing the betaine. Examples of betaines include trimethylammonium methylenyl, ethylenyl, propylenyl or phenyl carboxylate. Preferable betaines include trimethylammonium methylenyl, ethylenyl or propylenyl carboxylate.

An amidobetaine is a derivative of a betaine in which one of the quaternizing alkyl groups is replaced by an aminoalkyl group. The amine of the aminoalkyl group is converted to an amide by amidation with a fatty acid. The amidobetaine is a fattycarboxamido alkyldialkylammonium alkyl carboxylate of the formula

R¹CO-NHR²N⁺(R³)₂R⁴COO-

wherein R¹ is a fatty C12-C30 alkyl group, R² is a linear C2-C6 alkyl group, each R³ is methyl or ethyl and R⁴ is a C1-C4 alkyl group. Preferred amidobetaines include those made from fatty acid mixtures derived from natural oils such as cocamidopropyl betaine, oliveoil amidopropyl betaine, palmkernel amidopropyl betaine, palmoil amidopropyl betaine, cornoil amide propyl betaine.

The concentration of betaine and amidobetaine when included in the hair treatment composition according to the invention ranges from about 0.1 wt% to about 5 wt%, preferably about 0.1 wt% to about 3 wt%, more preferably about 0.1 wt% to about 1 wt% relative to the total weight of the composition.

### Optional Polymeric Film Formers, Rheologic Control Agents and Thickeners

Further optional additives as constituents for inclusion in the second component may also include preformed polymers such as a polyolefin, a polyester, a polyamide, a polyurethane, a poly urea and a copolymer thereof are useful as rheologic control and thickening additives and/or film forming additives for the general stand-alone conditioner, stand-alone leave-on conditioner, stand-alone rinse-off conditioner and stand alone mask. The preformed polymer at least in part contributes to rheology control and thickening of the composition as well as promotion of retention of the constituents of the hair treatment composition after rinsing, combing, toweling and drying. The preformed polymer may be formulated into the hair treatment composition at low to very low concentrations on the order of 0.01 to 3 wt%, preferably 0.1 to 1.5 wt%, more preferably 0.1 to 1 wt% relative to the total weight of the composition.

Included are a homopolymer and a copolymer containing ethylenically unsaturated monomers of the ester and/or amide type, as well as hydroxy alkyl ester monomers, diacid/diamine monomers and the like to form a polyester and/or a polyamide optionally and preferably with ester and/or amide pendant groups. Included are a homo- and copolymer of vinylpyrrolidone, polysaccharides, polyacrylamides, methyl methacrylate/ethylene glycol dimethacrylate copolymers, butyl methacrylate/methyl methacrylate copolymers, and polymethyl methacrylates, glucans, modified or unmodified starches (i.e. hydroxypropyl starch phosphate), amylose, amylopectin, glycogen, dextrans, celluloses and derivatives thereof (methylcelluloses, hydroxyalkylcelluloses, ethylhydroxyethylcelluloses), mannans, xylans, lignins, arabans, galactans, galacturonans, chitin, chitosans, glucoronoxylans, arabinoxylans, xyloglucans, glucomannans, pectic acids and pectins, arabinogalactans, carrageenans, agars, gums arabic, gums Tragacanth, Ghatti gums, Karaya gums, carob gums, galactomannans and mixtures thereof.

### Optional Coloring Constituents

Another optional, additional constituent for inclusion in the second component of the hair treatment composition includes direct dyes and henna dyes to provide coloring benefits. The technology of the present invention is suitable for henna dyes, nonionic direct dyes, especially for nonionic nitrobenzene derivatives and anionic direct dyes. The optional coloring constituents may be added to touch up hair color temporarily or semi-permanent between permanent dye applications such as by oxidative dye treatment. Typically, such conditioners containing coloration benefits are used to touch up hair roots, especially grey hair roots resulting from dyed hair that has grown.

Examples of nitrobenzene derivatives include but are not limited to, for example, 4-hydroxypropylamino-3-nitrophenol, 1-amino-5 chloro-4-[(2,3-dihydroxypropyl)amino]-2-nitrobenzene (HC Red No. 10), and 5-chloro-1,4-[di-(2,3-dihydroxypropyl)amino]-2-nitrobenZene (HC Red No. 11) or 4-hydroxypropylamino-3-nitrophenol. Nonionic nitro dyes useful herein include, for example, 1,4-bis-[(2-hydroxyethyl)amino]-2-nitrobenzene, 1 -(2-hydroxyethyl)amino-2-nitro-4-[di-(2-hydroxyethyl)amino]benzene (HC Blue No. 2), 1-amino-3-methyl-4-[(2-hydroxyethyl)amino]-6-nitrobenzene (HC Violet No. 1), 2 -amino -4 , 6 - dinitrophenol, 1 ,4-diamino-2-nitrobenzene (C176070), 4-amino-2-nitrodiphenylamine (HC Red No. 1), 1-amino-4-[di-(2-hydroxyethyl)amino]-2-nitrobenzenehydrochloride (HC Red No. 13), 1-amino-5-chloro-4-[(2-hydroxyethyl)amino] -2 -nitrobenzene, 4-[(2-hydroxyethyl)amino] -3 - nitrophenol, 4-[(2-nitrophenyl)amino]phenol (HC Orange No. 1), 1-[(2-amino ethyl)amino]-4-(2-hydroxyethoxy)-2-nitrobenzene (HC Orange No. 2), 4-(2,3-dihydroxypropoxy)-1-[(2-hydroxyethyl)amino]-2-nitrobenzene(HC Orange No. 3), 1-amino-2-[(2-hydroxyethyl)amino]-5 nitrobenzene (HC Yellow No. 5), 1 -(2 -hydroxyethoxy)-2-[(2 hydroxyethyl)amino]-5-nitrobenZene (HC Yellow No. 4), 1-[(2-hydroxyethyl)amino]-2-nitrobenZene (HC Yellow No. 2), 2- [(2-hydroxyethyl)amino] - 1 -methoxy-5 -nitrobenzene as well as similar variations and derivatives thereof as known in the dye literature.

Other nonionic direct dyes useful herein include, for example, 1,4-di-[(2,3 - dihydroxypropyl)amino]-9,10-anthraquinone, 1,4-di-[(2-hydroxyethyl)amino] -9,10-anthraquinone (C161545, Disperse Blue 23), 1-amino-4-hydroxy-9,10-anthraquinone (C160710, Disperse Red 15), 1 -hydroxy-4-[(4-methyl-2-sulfophenyl)amino]-9,10-anthraquinone, 7-beta-D-glucopyranosyl-9,10-dihydro-1 -methyl-9, 10-dioxo -3 ,5 ,6,8-tetrahydroxy-2-anthracenecarboxylic acid (C175470, Natural Red 4), 2-((4-(di-(2-hydroxyethyl)amino)phenyl)amino)-5-((2-hydroxyethyl)amino)-2,5-cyclohexadien-1,4-dione (HC Green No. 1), 2-hydroxy-1,4-naphthoquinone (C175480, Natural Orange No. 6), 1,2-dihydro-2-(1,3-dihydro-3-oxo-2H-indol-2-ylidene)-3H-indol-3-one (C173000), 1-[di-(2 - hydroxyethyl)amino] -4-[(4 -nitrophenyl)azo]benzene, (Disperse Black No. 9), 4-[(4-aminophenyl)azo]-1[di-(2-hydroxyethyl)amino]-3-methylbenZene (HC YellowNo. 7), 2), tri-(4-amino-3-methylphenyl) carbenium chloride (Cl42520; Basic Violet No. 2), 1-[(4-aminophenyl)azo]-7 (trimethylammonio)-2-naphthol chloride (Cl12250; Basic Brown No. 16), 3-[(4-amino-2,5-dimethoxyphenyl)azo]-N, N,N-trimethylbenzolaminium chloride (C11 12605, Basic Orange No. 69), 1-[(4-amino-2-nitrophenyl)azo]-7-(trimethylammonio)-2-naphthol chloride (Basic Brown No. 17), and 1-methyl-4-((methylphenyl-hydrazono)methyl)pyridinium methyl sulfate (Basic Yellow No. 87).

Other cationic direct dyes useful herein include, for example, Benzenamine, 4-[(2,6-Dichlorophenyl)(4-lmino-3,5 -Dimethyl-2,5-Cyclohexadien-1-ylidene)Methyl]-2,6 Dimethyl-, Phosphate) (HC Blue No. 15), 1-(2-morpholiniumpropylamino)-4-hydroxy- 9, 1 O-anthraquinone methylsulfate, and 1-[(3-(dimethylpropylaminium)propyl)amino]4-(methylamino)-9,10-anthraquinone chloride.

Anionic direct dyes useful herein include, for example, disodium bis[4-(N-ethyl-N-3-sulfonatophenylmethyl)aminophenyl]phenylmethylium (INCI name: Acid Blue 9), Benzenesulfonic acid, 2-[(9,10-Dihydro-4-hydroxy9,10-Dioxo-1-anthracenyl)amino]-5-methyl-, monosodiumsalt (INCI name: Ext. Violet 2), p-((2-Hydroxy-1-naphthyl)azo)benzenesulfonic acid sodium salt (INCI name: Orange 4), 2,2'-(1,4-Anthraquinonylenediimino)bis(5-methylbenzenesulfonic acid) disodium salt (INCI name: ACID GREEN 25), Acides 2-(2-quinoleyl) 1,3-indanedione mono, di, trisulfoniques, sodium salt (INCI name: Yellow 10), 5-amino-4-hydroxy-3-0-(phenylazo)-2,7-naphthalenesulfonic acid, disodium salt (INCI name: Acid Red 33).

The concentration of the optional dye constituent additive for the secondary component may range from about 0.001 wt% to about 5 wt%, preferably from about 0.001 wt% to about 2 wt% and/or a concentration of the optional dye constituent in the hair treatment composition that will provide an appropriate color touch-up of hair. The optional dye constituent may be formulated as a part of the hair treatment composition or may be provided as a separate additive in medium that may be combined with the hair treatment composition at time of use. Provision as a separate additive enables fine control of the dye constituent additive to the hair treatment composition and enables adjustment of the degree of color touch-up.

### Further Optional Constituents

The compositions of the present invention can also additionally comprise any other suitable constituents as optional additives for the second component as desired. For example, the composition can optionally include other active or inactive ingredients known for scalp health benefits.

In an embodiment of the present invention, a scalp health active may be added to provide scalp benefits. This group of scalp health materials is varied and provides a wide range of benefits including anti-dandruff, anti-fungal, anti-microbial, moisturization, barrier improvement, and anti-oxidant, anti-itch, and sensates. Such health actives include but are not limited to: zinc pyrithione, climbazole, octopirox, vitamin E and F, salicylic acid, phenoxyethanol, ethylhexyl glycerin, glycols, glycolic acid, PCA, PEGs, erythritol, glycerin, lactates, hyaluronates, allantoin and other ureas, betaines, sorbitol, glutamates, xylitols, menthol, menthyl lactate, isocyclomone, benzyl alcohol, and natural extracts/oils including peppermint, spearmint, argan, jojoba and aloe. The compositions may include other common hair ingredients. The CTFA Cosmetic Ingredient Handbook, Tenth Edition (published by the Cosmetic, Toiletry, and Fragrance Association, Inc., Washington, D.C.) (2004) (hereinafter "CTFA"), describes a wide variety of nonlimiting materials that can be added to the composition herein. Examples of these ingredient classes include but are not limited to: aesthetic components such as fragrances, pigments, colorings/colorants, essential oils, sensates, etcantifoaming agents, antimicrobial agents, biological additives, buffering agents, bulking agents, chelating agents, chemical additives, astringents, biocides, film formers or materials, pH adjusters, reducing agents, sequestrants, and anionic surfactants.

### Combinations of Primary Component and one or more of the constituents and/or embodiments of the Optional Second Component

Combinations of the primary component composed of the dual organic acid embodiments summarized above may be made with any one or more of the foregoing constituents and/or embodiments, and/or preferred embodiments and/or preferred constituents, and/or more preferred embodiments and/or more preferred constituents of the optional secondary component and additives for the secondary component as described above.

### Medium

The hair treatment composition of the present invention may comprise an aqueous carrier (including an all aqueous medium and an aqueous organic medium) as a cosmetically acceptable medium. Accordingly, embodiments of the compositions of the present invention can be in the form of pourable liquids (under ambient conditions). Such compositions will therefore typically comprise an aqueous carrier, which may be present at a level of from about 20 wt. % to about 95 wt. %, or from about 60 wt. % to about 85-90 wt. %. The aqueous carrier may comprise water, or a miscible mixture of water and organic solvent, and in one aspect may comprise water with minimal or no significant concentrations of organic solvent, except as otherwise incidentally incorporated into the composition as minor ingredients of other components.

The aqueous carrier useful in the present invention may comprise water solutions of lower alkyl alcohols and polyhydric alcohols. The lower alkyl alcohols useful herein are monohydric alcohols having 1 to 6 carbons, in one aspect, ethanol and isopropanol. The polyhydric alcohols useful herein include propylene glycol, hexylene glycol, glycerin, and propane diol. Aqueous-organic carriers for the hair treatment compositions of the invention may comprise from about 50 wt% to about 99 wt% water, preferably about 75 wt% to about 95 wt% water, more preferably about 85 wt% to about 95 wt% water with the remainder being a mono or polyhydric alcohol, preferably any one or a mixture of any two or more of ethanol, isopropanol, propylene glycol and glycerin.

### pH of the Composition

Embodiments of the present invention may optionally incorporate not only the organic acid constituents but also organic acidifying and basifying agents such as citric acid, acetic acid, hydrochloric acid and alkali metal hydroxides such as sodium or potassium hydroxide. Additionally, buffering agents such as carbonate, bicarbonate, acetate, citrate, biphosphate, and/or borate compounds may be included. These agents enable balance of the pH of the hair treatment composition of the present invention so that it is not an irritant to humans such as human scalp and/or face. Consequently, the concentration of the acidifying, basifying and/or buffering agent may be adjusted so that the pH is in the range of about 3 to about 9, preferably in a range of about 4 to about 8.

### Method of Preparation of Hair Treatment Composition

The method for preparation of the hair treatment composition spans techniques from facile combination and mixing of all constituents with the desired aqueous-organic or aqueous medium to sequential combination of hydrophobic and hydrophilic constituents in appropriate solvents followed by combination of these separately formed mixtures. When all constituents are soluble in the desired medium and addition of one of the constituents does not cause separation and/or precipitation of other constituents already present, the method for preparation may involve formation of the appropriate, desired medium followed by addition and mixing of each separate constituent at the desired weight percentage concentrations. If some of the constituents are highly hydrophobic, such as the fatty alcohols and the like, the preparation method involves formation of an emulsion of the hydrophobic constituents and surfactants such as the cationic and nonionic surfactants in the desired medium. The emulsion may be slowly added to a mixture of the hydrophilic constituents in medium and additional surfactant may be added to maintain the emulsified mixture. When the hydrophobic constituents are solids, they can be melted, combined with nonionic surfactant and a portion of the warmed medium in which the hydrophobic constituents are soluble and/or to form an emulsion. The solution and/or emulsion can then be cooled and added slowly to the preformed solution of hydrophilic constituents in the medium while vigorously stirring to maintain the solution and/or emulsion. If micro-particulates are to be included in the composition, they may be slowly added to the formed solution and/or emulsion as a mixture with medium and suspending agent.

The preparation of the hair treatment composition as a general stand-alone conditioner, a leave-on stand-alone conditioner, a rinse-off stand-alone conditioner or a stand-alone mask may be formulated as a hair gel, hair paste, hair cream, hair rinse, hair oil, hair pomade, hair foam, hair spray, hair aerosol, hair tonic and/or hair lotion. These uses of the hair treatment composition may be perfected by adjusting the concentrations of primary and secondary components and medium to provide the appropriate viscosities and flowabilities of the resulting formulations to provide the hair gel, hair paste, hair cream, hair rinse, hair oil, hair pomade, hair foam, hair spray, hair aerosol, hair tonic and/or hair lotion.

### Kit for Cleaning and Conditioning Regimen

The kit for practice of the cleaning and conditioning regimen includes unit containers of a cleansing and cleaning composition (shampoo) and a hair treatment composition (the stand-alone conditioners). The shampoo for the unit container comprises one or more of an anionic surfactant such as a branched and/or linear C8 to C20 alkyl and/or alkenyl sulfate, a linear and/or branched C8 to C20 alkyl benzene sulfate or a linear and/or branched C8 to C20 alkyl benzyl sulfate or the corresponding ether and/or glyceryl and/or sugar alcohol extensions of the alkyl, alkenyl alkyl benzene and/or alkyl benzyl sulfates in which an alkyl, alkyl benzene or alkyl benzyl alcohol is coupled to the extension which in turn is coupled to sulfate. The extensions may be obtained from ethylene epoxide/ethylene glycol, propylene epoxide/1,2-propylene glycol, glycerin and/or sugar alcohol monomers. Examples include but are not limited to lauryl sulfate, laureth sulfate, cocoyl sulfate, coceth sulfate, tridecyl benzene sulfate tridecyl benzyl sulfate. The counter ions may be sodium and/or potassium.

The shampoo for the unit container may include solely or in combination with the sulfate anionic surfactant, a non-sulfate anionic surfactant and/or a non-ionic surfactant as described above. The non-sulfate anionic surfactant may be selected from the group of an acyl isethionate/methyl isethionate, acyl glycinate, acyl taurate, acyl amino acid, acyl sarcosinate, sulfosuccinate and sulfonate, wherein the acyl groups of all these surfactant classes comprise from 6 to 30 carbon atoms.

The shampoo for the unit container may also contain conditioning agents such as but not limited to one or more of a cationic surfactant, a silicone and/or a cationic polymer as well as an anti-dandruff agent.

The unit container of the hair treatment composition includes the general stand-alone conditioner, the stand-alone leave-on conditioner or the stand-alone rinse-off conditioner.

### Method of Application

The general purpose stand-alone conditioner, stand-alone leave-on conditioner, stand-alone rinse-off conditioner and stand-alone mask embodiments of the hair treatment composition may be applied neet to anagenic hair (hair on the head of a human) or the stand-alone general purpose conditioner or the stand-alone rinse-off conditioner may be diluted with water or a combination of water and a water miscible alcohol and applied to anagenic hair. After application, these embodiments of the hair treatment composition on the hair may be massaged and worked through the hair to distribute the embodiments throughout the hair. Following a sufficient time to accomplish conditioning, and/or further functions of the hair treatment composition on the hair, the treatment may be further handled according to the embodiment of hair treatment composition, i.e. conditioner or mask used. If the embodiment is the general purpose stand-alone conditioner, the hair may optionally be rinsed with water. If the hair treated with this embodiment is not rinsed, especially if the embodiment has been diluted with water either before its application to the hair or during a bath or shower, the hair may be toweled to remove excess conditioner and then dried as described below. If the embodiment is the stand-alone rinse-off conditioner, the hair may be rinsed with water and toweled to remove rinse water. In both of these embodiment treatments, the hair may then be optionally combed followed by drying the conditioned hair with ambient blown or warmed air. If the hair treatment composition is a leave-on conditioner or mask, after working the leave-on conditioner or mask throughout the hair, the hair may be combed and/or brushed and/or toweled briefly to remove excess composition if any and allowed to dry naturally preferably without use of blown air.

One or more of the general stand-alone conditioner, the stand-alone, leave-on conditioner, the stand-alone or rinse-off conditioner also may form a second part of a shampoo and conditioning regimen. The regimen is practiced with the above described kit of separate containers of cleansing and cleaning composition (shampoo) and conditioner. The regimen involves a first step of shampooing the hair with a cleansing and cleaning composition containing a sulfate and/or non-sulfate anionic surfactant such as Wella Professionals Invigo^{®} Shampoo, Wella Professionals Fusion Intense Repair^{®} Shampoo or Wella Professionals Elements Renewing^{®} Shampoo followed by rinsing and optionally towel drying to dampness. The second part of the regimen follows the foregoing treatment process with one of the stand-alone conditioners.

Use of the stand-alone mask typically does not follow a shampoo regimen but may be applied to shampooed, rinsed and dried hair.

It is believed that the regimen procedure using the two unit kit for cleaning and conditioning delivers a better conditioning result than will use of a single shampoo composition also containing conditioning agents. As discussed above, the anionic surfactant detergent of a cleansing and cleaning composition such as a shampoo removes the natural oils, fatty acids and natural smoothing constituents from hair surfaces. It is believed that conditioning agents such as fatty acids, cationic surfactants and the like present in a single combination cleansing, cleaning and conditioning composition containing anionic surfactant detergents and conditioning agents will be largely to substantially eliminate and/or remove from the hair surfaces the added conditioning agents as the shampoo composition is foamed and scrubbed onto the hair, is repeatedly diluted with water, rinsed and/or especially when the hair is subsequently washed and/or contacted with body soap and/or body wash while bathing and/or showering. Although conditioning shampoos may be formulated to develop and deposit on the hair a coacervate conditioner of an anionic surfactant and a cationic surfactant during the shampoo process, the coacervate may be dissolved and/or removed and/or scrubbed off, and/or lessened by repeated shampooing, rinsing and washing with cleaning compositions typically and usually applied during a shower and/or bath, including but not limited to anionic surfactant and/or soap not containing cationic surfactants, body wash, bubble bath salts, skin smoothing agents and the like. For these reasons, the regimen for shampooing and conditioning delivers desirable conditioning results to hair.

### EXAMPLES

The following examples illustrate the present invention. The exemplified compositions can be prepared by conventional formulation and mixing techniques. It will be appreciated that other modifications of the present invention within the skill of those in the hair care formulation art can be undertaken without departing from the spirit and scope of this invention. All parts, percentages, and ratios herein are by weight unless otherwise specified. Some components may come from suppliers as dilute solutions.

A surprisingly strong hair conditioning enhancement has been found for the combination of a dicarboxylic acid with a fatty monocarboxylic acid. The strong hair conditioning enhancement synergy of the dicarboxylic acid/fatty monocarboxylic acid combination was found in both rinse-off and also leave-on product applications. The following samples were evaluated with regards to its conditioning performance:

**Table 1 - Test samples (rinse-off conditioners/masks)**

| **Raw Material Name** | **Test sample 1** | **Test sample 2** | **Test sample 3** | **Test sample 4** | **Test sample 5** |
|---|---|---|---|---|---|
| Cetyl Alcohol | 8.0 g | 8.0 g | 8.0 g | 8.0 g | 8.0 g |
| Paraffinum Liquidum | 3.0 g | 3.0 g | 3.0 g | 3.0 g | 3.0 g |
| Isopropyl Myristate* | 3.0 g | 3.0 g | 3.0 g | 3.0 g | 3.0 g |
| Cetrimonium Chloride | 1.0 g | 1.0 g | 1.0 g | 1.0 g | 1.0 g |
| Behentrimonium Chloride | 0.4 g | 0.4 g | 0.4 g | 0.4 g | 0.4 g |
| Amodimethicone | 0.3 g | 0.3 g | 0.3 g | 0.3 g | 0.3 g |
| Ceteareth-25 | 1.5 g | 1.5 g | 1.5 g | 1.5 g | 1.5 g |
| Coco-Glucoside | 0.45 g | 0.45 g | 0.45 g | 0.45 g | 0.45 g |
| Polyquaternium-37 | 0.125 g | 0.125 g | 0.125 g | 0.125 g | 0.125 g |
| Oleic Acid | - | 0.1 g | - | 0.1 g | 0.5 g |
| Malic Acid | - | - | 3.0 g | 3.0 g | 3.0 g |
| Sodium Hydroxide | 1.0 g | 1.0 g | 1.0 g | 1.0 g | 1.0 g |
| Disodium EDTA | 0.1 g | 0.1 g | 0.1 g | 0.1 g | 0.1 g |
| Phenoxyethanol | 0.9 g | 0.9 g | 0.9 g | 0.9 g | 0.9 g |
| Ethylhexylglycerin | 0.1 g | 0.1 g | 0.1 g | 0.1 g | 0.1 g |
| Perfume | 0.3 g | 0.3 g | 0.3 g | 0.3 g | 0.3 g |
| Water | to 100 g | to 100 g | to 100 g | to 100 g | to 100 g |

| | | | | | |
|---|---|---|---|---|---|
| • A fatty acid ester; other similar esters include isopropyl palmitate, glyceryl monooleate | | | | | |

**Table 2 - Test samples: leave-on conditioner**

| **Raw Material Name** | **Test sample 6** | **Test sample 7** | **Test sample 8** | **Test sample 9** | **Test sample 10** |
|---|---|---|---|---|---|
| Isohexadecane | 13.2 | 13.2 | 13.2 | 13.2 | 13.2 |
| ISOPROPYL PALMITATE | 11 | 11 | 11 | 11 | 11 |
| Stearyl Alcohol | 0.5 | 0.5 | 0.5 | 0.5 | 0.5 |
| Steareth-2 | 0.18 | 0.18 | 0.18 | 0.18 | 0.18 |
| SODIUM BENZOATE | 0.4 | 0.4 | 0.4 | 0.4 | 0.4 |
| STEARETH-21 | 0.32 | 0.32 | 0.32 | 0.32 | 0.32 |
| PEG 23M | 0.2 | 0.2 | 0.2 | 0.2 | 0.2 |
| Disodium EDTA | 0.05 | 0.05 | 0.05 | 0.05 | 0.05 |
| Polyacrylamide 40% solution | 2.5 | 2.5 | 2.5 | 2.5 | 2.5 |
| 10.000cSt Dimethicone | 1.5 | 1.5 | 1.5 | 1.5 | 1.5 |
| Benzyl Alcohol | 0.4 | 0.4 | 0.4 | 0.4 | 0.4 |
| Phenoxyethanol | 0.4 | 0.4 | 0.4 | 0.4 | 0.4 |
| Histidine | 0.015 | 0.015 | 0.015 | 0.015 | 0.015 |
| Malic Acid | - | - | 0.3 | 0.3 | 0.3 |
| Oleic Acid | - | 0.1 | - | 0.1 | 0.5 |
| Sodium Hydroxide | 0.15 | 0.15 | 0.15 | 0.15 | 0.15 |
| PANTHENOL 50% solution | 0.536 | 0.536 | 0.536 | 0.536 | 0.536 |
| Vitamin E Acetate | 0.2 | 0.2 | 0.2 | 0.2 | 0.2 |
| Fragrance | 0.6 | 0.6 | 0.6 | 0.6 | 0.6 |
| Water | to 100 g | to 100 g | to 100 g | to 100 g | to 100 g |

### Test methodology: Combing Force Measurement

The ease of combing of hair is a major parameter for describing the quality/degree of damage of hair on the one hand and/or the effectiveness of conditioning respectively hair repair treatments on the other hand.

The principle of most methods for the determination of combing properties consists in measuring the force to pull a comb through a hair tress under definite conditions (Ref.: C.R. Robbins, Chemical and Physical Behavior of Human Hair, 5th Edition, p. 646 ff., Springer-Verlag, Berlin Heidelberg 2012. ISBN 978-3-642-25610-3).

For this test, an automated device is used in which the tresses to be tested are taken from a storage chamber and fixed to a force meter. The tresses are combed automatically at constant speed. For each combing stroke, the combing force is recorded as a function of the distance combed through (length of the tress). For comparison purposes, 20 combing strokes per tress on at least 3 tresses per product sample are averaged.

The Combing Force is the average force along the tress. The lower the combing force the better the combing ease/smoothness.

### Hair tresses preparation for the combing force measurements

Hair tresses (Caucasian hair), weight 2g, 17cm long, medium-blonde color, are two times bleached to simulate hair damage and then soaked in city water for 10 min (35°C ± 2°C). Afterwards, the tresses are adjusted to 50% rel. humidity by weight, then combed twice (rough & fine side of comb).

Rinse-off conditioner/mask application: 0.25ml/g hair of the hair care composition to be tested are applied to the tresses and massaged in for 1 min. The tresses are rinsed off for 1 min using city water (6L/min; 35°C ± 2°C). Afterwards, the tresses are adjusted to 50% rel. humidity by weight. The tresses are combed twice (rough & fine side of comb), are left hanging overnight in the climatic chamber (20°C, 65% rel. humidity), again combed twice and combing measurement is carried out.

Leave-on conditioner application: 0.1ml/g hair of the leave-on treatment are applied to the tresses and massaged in for 1 min. Afterwards, the tresses are adjusted to 50% rel. humidity by weight. The tresses are combed twice (rough & fine side of comb), are left hanging overnight in the climatic chamber (20°C, 65% rel. humidity), again combed twice and combing measurement is carried out.

### Examination results

Tables 3 and 4 show the measured wet combing forces in Newton (N) of hair swatches subjected to damage and then treated with the hair treatment compositions according to Table 1 and Table 2. The treated hair swatches subjected to the mechanical combing measurement described above include damaged hair (two times bleached) with no conditioning treatment as reference and damaged hair (two times bleached) treated with test samples 1- 10.

**Table 3 - Combing force results rinse-off conditioner/mask samples 1-5**

| **Combing Force Average / N** | **Combing Force Standard Deviation / N** | **n (number of combing tests per sample)** | **Sample** |
|---|---|---|---|
| 0.856 1,2,4,5 | 0.087 | 3 | R) Reference, 2x bleached (damaged) hair |
| 0.562 ^{R,5} | 0.118 | 3 | 1) 2x bleached hair + treated with test sample 1 - rinse-off conditioner/mask with NO fatty monocarboxylic acid + NO dicarboxylic acid |
| 0.704 ^{R,4,5} | 0.025 | 3 | 2) 2x bleached hair + treated with test sample 2 - rinse-off conditioner/mask with 0.1% fatty monocarboxylic acid (oleic acid) +NO dicarboxylic acid |
| 0.631 ⁵ | 0.147 | 3 | 3) 2x bleached hair + treated with test sample 3 - rinse-off conditioner/mask with NO fatty monocarboxylic acid + 3% dicarboxylic acid (malic acid) |
| 0.460 ^{R,2,5} | 0.037 | 3 | 4) 2x bleached hair + treated with test sample 4 - rinse-off conditioner/mask with 0.1% fatty monocarboxylic acid (oleic acid) + 3% dicarboxylic acid (malic acid) |
| 0.331 ^{R,1,2,3,4} | 0.022 | 3 | 5) 2x bleached hair + treated with test sample 5 - rinse-off conditioner/mask with 0.5% fatty monocarboxylic acid (oleic acid) + 3% dicarboxylic acid (malic acid) |

| | | | |
|---|---|---|---|
| Superscript index indicates statistically significant differences (t-test, p ≥ 0.95) | | | |

**Table 4 - Combing force results leave-on conditioner samples 6-10**

| **Combing Force Average / N** | **Combing Force Standard Deviation / N** | **n (number of combing tests per sample)** | **Sample** |
|---|---|---|---|
| 0.636 ⁵ | 0.139 | 3 | R) Reference, 2x bleached (damaged) hair |
| 0.635 ^{2,4,5} | 0.026 | 3 | 6) 2x bleached hair + treated with test sample 6 - leave on conditioner with NO fatty monocarboxylic acid + NO dicarboxylic acid |
| 0.685 ^{1,4,5} | 0.014 | 3 | 7) 2x bleached hair + treated with test sample 7 - leave on conditioner with 0.1% fatty monocarboxylic acid (oleic acid) +NO dicarboxylic acid |
| 0.662 ⁵ | 0.116 | 3 | 8) 2x bleached hair + treated with test sample 8 - leave-on conditioner with NO fatty monocarboxylic acid + 3% dicarboxylic acid (malic acid) |
| 0.485 ^{1,2,5} | 0.062 | 3 | 9) 2x bleached hair + treated with test sample 9 - leave-on conditioner with 0.1% fatty monocarboxylic acid (oleic acid) + 3% dicarboxylic acid (malic acid) |
| 0.362 ^{R,1,2,3,4} | 0.038 | 3 | 10) 2x bleached hair + treated with test sample 10 - leave on conditioner with 0.5% fatty monocarboxylic acid (oleic acid) + 3% dicarboxylic acid (malic acid) |

| | | | |
|---|---|---|---|
| Superscript index indicates statistically significant differences (t-test, p ≥ 0.95) | | | |

The results indicate clearly that hair treated with a rinse-off conditioner/mask or a leave on treatment compositions containing both fatty monocarboxylic acid (oleic acid) AND a dicarboxylic acid (malic acid) (samples 4 and 5) shows better combing (lower combing forces) versus those treated with rinse-off conditioner/masks or leave on treatments containing only dicarboxylic acid but no fatty monocarboxylic acid (sample 3) or only fatty monocarboxylic acid but no dicarboxylic acid (sample 2).

In fact, fatty monocarboxylic acid or dicarboxylic acid alone do not improve the conditioning performance of a rinse conditioner mask or leave on treatment. Thus, the combination of fatty monocarboxylic acid and dicarboxylic acid shows a synergistic effect on combability respectively hair repair effectiveness.

### EXAMPLES WITH OPTIONAL SECOND COMPONENT INGREDIENTS

Additional examples are prepared to show combing force results relative to variations of the secondary components present in the hair treatment compositions of the primary component. The variations are of the hydrocarbon and fatty acid ester added to the primary component of dicarboxylic acid, fatty carboxylic acid.

The following examples illustrate the benefits of stand-alone conditioners with at least the dicarboxylic acid and fatty carboxylic acid and the enhancement of benefits by addition of one or more of the lipophilic constituents of the second component.

The following examples 11-12 are prepared by melting the fatty alcohols, hydrocarbons, and fatty acid esters, adding organic solvent, a portion of water and nonionic surfactants such as cetereth-25 and coco glucoside to form an emulsion, followed be addition of the water soluble ingredients and the remaining water and vigorous stirring to form a homogeneous mixture of the conditioner/mask embodiments of the hair treatment composition of the invention.

### Example 11 (leave-on treatment with primary component, hydrocarbon and fatty acid ester)

| **Raw Material** | **Amount** |
|---|---|
| Propylenglycol | 3.0 g |
| Amodimethicone | 1.3 g |
| Quaternium-80 | 1.0 g |
| Cetrimonium Chloride | 1.0 g |
| Behentrimonium Chloride | 1.6 g |
| Amodimethicone | 0.3 g |
| D,L-Tocopheryl Acetate | 0.2 g |
| Glyceryl Monooleate | 0.25 g |
| Polyquaternium-37 | 0.125 g |
| Oleic Acid | 0.1 g |
| Malic Acid | 0.3 g |
| Sodium Hydroxide | 0.1 g |
| Disodium EDTA | 0.1 g |
| Squalane | 0.1 g |
| Phenoxyethanol | 0.1 g |
| Methylparaben | 0.2 g |
| Perfume | 0.3 g |
| Water | to 100 g |

### Example 12 (leave-on treatment without lipids, fatty acid and dicarboxylic acid

| **Raw Material** | **Amount** |
|---|---|
| Propylenglycol | 3.0 g |
| Amodimethicone | 1.3 g |
| Quaternium-80 | 1.0 g |
| Cetrimonium Chloride | 1.0 g |
| Behentrimonium Chloride | 1.6 g |
| Amodimethicone | 0.3 g |
| D,L-Tocopheryl Acetate | 0.2 g |
| Polyquaternium-37 | 0.125 g |
| Sodium Hydroxide | 0.1 g |
| Disodium EDTA | 0.1 g |
| Phenoxyethanol | 0.1 g |
| Methylparaben | 0.2 g |
| Perfume | 0.3 g |
| Water | to 100 g |

### Combing Force Measurement

The procedure for combing force measurement follows the description provided above for Examples 1-10.

### Hair tresses preparation for the combing force measurements examples 11-12

Hair tresses (Caucasian hair), weight 2g, 17 cm long, medium-blonde color, are two times bleached to simulate hair damage and then soaked in city water for 10 min (35°C ± 2°C). Afterwards, the tresses are adjusted to 50% rel. humidity by weight, then combed twice (rough & fine side of comb). 0.25ml/g hair of the hair care composition to be tested are applied to the tresses and massaged in for 1 min. The tresses are rinsed off for 1 min using city water (6L/min; 35°C ± 2°C). Afterwards, the tresses are adjusted to 50% rel. humidity by weight. The tresses are combed twice (rough & fine side of comb) and placed inside a wet magazine of the combing device storage chamber for the actual wet combing force measurements.

### Examination results

The following table shows the measured wet combing forces in Newton (N) of hair swatches subjected to damage and then treated with the hair treatment composition according to the invention as well as treatment compositions lacking one or more of the four components of the hair treatment composition of the invention. The treated hair swatches subjected to the mechanical combing measurement described above include:
a) virgin hair,
b) damaged hair (two times bleached),
c) damaged hair (two times bleached) treated with a liquid composition of example 11 including the primary and secondary components of the hair treatment composition: dicarboxylic acid, fatty acid, fatty acid ester, hydrocarbon),
d) damaged hair (two times bleached) treated with a liquid composition containing the ingredients of example 11 except that this composition does not contain the lipids, fatty acid and dicarboxylic acid of example 11; this is example 12.

| **Combing Force / N** | **Sample** |
|---|---|
| 0.125 | a) Untreated (virgin hair) |
| 0.293 | b) 2x bleached (damaged hair) |
| 0.073 | c) 2x bleached + treated with example 11 (composition with lipids, fatty acid and dicarboxylic acid) |
| 0.087 | f) 2x bleached + treated with example 12 (composition without lipids, without fatty acid and without dicarboxylic acid) |

The results indicate that hair treated with compositions containing lipid plus fatty acid and dicarboxylic acid (sample c, example 11) show a better combing (lower combing forces) versus the composition with a leave on conditioner composition without the primary component or any of the constituents of the secondary component. These combing results are not comparable with the combing results of examples 1-10 because of the use of different hair swatches and composition ingredients.

### STATEMENTS OF EMBODIMENTS OF THE INVENTION

The following statements of embodiments of the invention describe aspects, features and parameters of the methods, compositions, and treatments according to the invention. These statements provide further disclosure of these aspects, features and parameters and may serve as claims of the invention.
1. A hair treatment composition comprising a general stand-alone conditioner, a stand-alone leave-on conditioner, a stand-alone rinse-off conditioner or a stand-alone mask comprising a cosmetically acceptable aqueous or aqueous-organic medium and at least a primary component comprising:
   a) a dicarboxylic acid comprising:
      1) a C3-C6 saturated, linear dicarboxylic acid with one hydroxyl group or a salt thereof; or
      2) a C3-C8 saturated, linear dicarboxylic acid with two or three hydroxyl groups or a salt thereof; or
      3) a C2-C13 saturated, linear dicarboxylic acid with or without one or more hydroxyl groups or
      4) any combination of 1), 2) or 3); and,
   b) a fatty monocarboxylic acid comprising:
      1) a C16-C18 linear, mono-unsaturated fatty monocarboxylic acid or salt thereof; or
      2) a C10 - C30 linear mono or multi-unsaturated fatty acid or salt thereof, or
      3) a C10 - C30 linear saturated fatty monocarboxylic acid, or
      4) any combination of 1), 2) or 3);
   wherein,
   the concentration range of the dicarboxylic acid of the primary component comprises about 0.01 wt% to about 35 wt%, and the concentration range of the fatty monocarboxylic acid of the primary component comprises about 0.01 wt% to about 35 wt%; and,
   the wt percentage ratio of dicarboxylic acid to fatty monocarboxylic acid of the primary component comprises about 50:1 to about 1:10, preferably about 30:1 to about 1:5; and,
   the concentrations of the dicarboxylic acid and the fatty monocarboxylic acid are relative to the total weight of the composition.
2. The hair treatment composition according to statement 1 wherein the weight percentage range of the dicarboxylic acid comprises about 0.01 wt% to about 35 wt%, or preferably about 0.1 wt% to about 25 wt%, or especially preferably from about 0.1 wt% to about 20 wt%, or more preferably from about 0.1 wt% to about 15 wt%, with exemplary concentrations of 0.1, 0.5. 1.0, 1.5, 2, 2.5, 3, 3.5, 4, 4.5, 5, 5.5, 6, 6.5, 7, 7.5, 8, 8.5, 9, 9.5 and 10 wt%'s and all digital integrals between these numbers; and the concentration range of the fatty monocarboxylic acid comprises about 0.01 wt% to about 35 wt%, or preferably about 0.015 wt% to about 25 wt%, or more preferably about 0.015 wt% to about 20 wt%, or especially more preferably about 0.015 wt% to about 15 wt%, with exemplary concentrations of 0.01, 0.015, 0.03, 0.1, 0.2, 0.5. 0.75, 1.0, 1.5, 2, 2.5, 3, 3.5, 4, 4.5, 5, 5.5, 6, 6.5, 7, 7.5, 8, 8.5, 9, 9.5 and 10 wt%'s and all digital integrals between these numbers; and the concentrations of the dicarboxylic acid and the fatty monocarboxylic acid being relative to the total weight of the composition.
3. The hair treatment composition according to any of the preceding statements further comprising a secondary component comprising a fatty acid ester and/or a linear or branched hydrocarbon wherein
   a) the fatty acid ester comprises
      1) an unsaturated C10 - C30 fatty acid with 1 or 2 unsaturations esterified with C1-C10 mono, di or tri alcohol; or
      2) a saturated C10 - C30 fatty acid esterified with C1-C10 mono, di or tri alcohol; or
      3) a combination of 1) and 2); and,
   b) the linear or branched hydrocarbon comprises
      1) a C10 - C40 hydrocarbon all saturated; or
      2) a C15 - C40 hydrocarbon with from 3 unsaturations to 6 unsaturations
      3) any combination of any of 1) and/or 2)
4. The hair treatment composition according to statement 3 wherein the concentration of the secondary component is about 0.0 wt% or the secondary component has a weight percentage concentration comprising from about 0.01 wt% to about 40 wt%, preferably up to about 35 wt%, more preferably up to about 30 wt%, especially more preferably up to about 25 wt%, most preferably up to about 20 wt%, especially most preferably up to about 15 wt% and all incremental wt%'s therebetween, all concentrations being relative to the total weight of the composition
5. The hair treatment composition according to any of the preceding statements wherein the dicarboxylic acid comprises a linear dicarboxylic acid with one, two, three or four hydroxyl groups.
6. The hair treatment composition according to any of the preceding statements wherein the dicarboxylic acid comprises at least a linear dicarboxylic acid with one or two hydroxyl groups.
7. The hair treatment composition according to any of the preceding statements wherein the dicarboxylic acid comprises at least a linear dicarboxylic acid with two or more hydroxyl groups.
8. The hair treatment composition according to any of the preceding statements wherein the fatty monocarboxylic acid comprises at least a saturated linear fatty monocarboxylic acid.
9. The hair treatment composition according to any of the preceding statements wherein the fatty monocarboxylic acid comprises at least an unsaturated linear fatty monocarboxylic acid with one, two or three olefinic unsaturations.
10. The hair treatment composition according to any of the preceding statements wherein the fatty monocarboxylic acid comprises at least an unsaturated linear fatty monocarboxylic acid with one or two, preferably one olefinic unsaturation.
11. The hair treatment composition according to any of the preceding statements wherein the dicarboxylic acid is selected from the group consisting of:
   a1) a linear organic C2-C13 dicarboxylic acid or a cosmetically acceptable salt thereof;
   a2) a linear organic C3-C13 dicarboxylic acid substituted with from 1 to 6 hydroxyl groups or a cosmetically acceptable salt thereof;
   a3) any combination of a1) - a2);
   and the fatty monocarboxylic acid is selected from the group consisting of:
   b1) a saturated linear organic C10-C30 fatty monocarboxylic acid or a cosmetically acceptable salt thereof;
   b2) an unsaturated linear organic C10-C30 fatty monocarboxylic acid with from 1 to 3 olefinic unsaturations or a cosmetically acceptable salt thereof; and
   b3) any combination of b1and b2.
12. The hair treatment composition according to any of the preceding statements wherein
   the saturated dicarboxylic acid comprises a C3 - C8 dicarboxylic acid with one or two hydroxyl groups, preferably a saturated C3 to C6 dicarboxylic acid with one hydroxyl group; and
   the fatty monocarboxylic acid is a monounsaturated C16 to C26 fatty monocarboxylic acid, more preferably a C16 to C22 monounsaturated monocarboxylic acid, especially more preferably a C16-C18 monounsaturated monocarboxylic acid.
13. The hair treatment composition according to any of the preceding statements wherein the dicarboxylic acid comprises one or more of oxalic acid, malonic acid, succinic acid, glutaric acid, adipic acid, brassylic acid, malic acid, hydroxysuccinic acid, tartaric acid, hydroxyglutaric acid, galactaric acid, salts thereof, or any combination thereof.
14. The hair treatment composition according to any of the preceding statements wherein the dicarboxylic acid comprises one or more of malonic acid, succinic acid, malic acid, hydroxysuccinic acid, tartaric acid, salts thereof, or any combination thereof.
15. The hair treatment composition according to any of the preceding statements wherein the dicarboxylic acid is:
   a) a C3-C6 dicarboxylic acid with one hydroxyl group or a salt thereof; or
   b) a C4-C6 dicarboxylic acid with two hydroxyl groups or a salt thereof; or
   c) any combination of a) and b).
16. The hair treatment composition according to any of the preceding statements wherein the dicarboxylic acid is a C3-C6 dicarboxylic acid with one hydroxyl group or a salt thereof.
17. The hair treatment composition according to any of the preceding statements wherein the dicarboxylic acid is malic acid, hydroxysuccinic acid, tartaric acid, salts thereof, or any combination thereof.
18. The hair treatment composition according to any of the preceding statements wherein the fatty acid is a linear saturated or unsaturated C10-C30 fatty monocarboxylic acid or a salt thereof.
19. The hair treatment composition according to any of the preceding statements wherein the fatty acid is a linear, unsaturated C10-C30 fatty monocarboxylic acid or a salt thereof.
20. The hair treatment composition according to any of the preceding statements wherein the fatty acid is a linear, unsaturated C10-C26 fatty monocarboxylic acid or a salt thereof.
21. The hair treatment composition according to any of the preceding statements wherein the fatty acid is myristoleic acid, palmitoleic acid, sapienic acid, oleic acid, elaidic acid, vaccenic acid, linoleic acid, linoelaidic acid, linolenic acid, arachidonic acid, eicosapentaenoic acid, erucic acid, a salt thereof, or any combination thereof.
22. The hair treatment composition according to any of the preceding statements wherein the fatty acid is myristoleic acid, palmitoleic acid, sapienic acid, oleic acid, elaidic acid, vaccenic acid, linoleic acid, linoelaidic acid, erucic acid, a salt thereof, or any combination thereof.
23. The hair treatment composition according to any of the preceding statements wherein the fatty acid is palmitoleic acid, sapienic acid, oleic acid, elaidic acid, vaccenic acid, linoleic acid, linelaidic acid, a salt thereof, or any combination thereof.
24. The hair treatment composition according to any of the preceding statements wherein the fatty acid is palmitoleic acid, sapienic acid, oleic acid or elaidic acid, a salt thereof, or any combination thereof.
25. The hair treatment composition according to any of the preceding statements wherein the fatty acid is sapienic acid, oleic acid, a salt thereof, or any combination thereof.
26. The hair treatment composition according to any of the preceding statements wherein the dicarboxylic acid is selected from malic acid, hydroxysuccinic acid, tartaric acid, a salt thereof, or any combination thereof and the fatty acid is selected from oleic acid, sapienic acid, palmitoleic acid, a salt thereof or mixtures thereof.
27. The hair treatment composition according to any of the preceding statements wherein the dicarboxylic acid is selected from malic acid or a salt thereof, or tartaric acid or a salt thereof, or any mixture thereof, and the fatty acid is selected from oleic acid or a salt thereof, palmitoleic/sapienic acid or a salt thereof, or any mixture thereof.
28. The hair treatment composition according to any of the preceding statements wherein the dicarboxylic acid is selected from malic acid or a salt thereof, and the fatty acid is selected from oleic acid or a salt thereof.
29. The hair treatment composition according to any of the preceding statements comprising a secondary component of at least a fatty ester and/or a hydrocarbon wherein each is present at a weight percentage concentration range of from about 0.1 wt% to about 10 wt% relative to the total weight of the composition.
30. A hair treatment composition according to any of the preceding statements wherein the secondary component comprises at least a fatty acid ester.
31. The hair treatment composition according to any of the preceding statements wherein the secondary component comprises at least the fatty acid ester wherein the esterified fatty acid comprises at least a fatty monocarboxylic acid of any of the preceding statements or any combination thereof and the esterifying alcohol comprises a C2 to C10 diol which is monoesterified or di-esterified by the fatty monocarboxylic acid or a C3-C6 triol which is mono, di or tri-esterified by the fatty monocarboxylic acid.
32. The hair treatment composition according to any of the preceding statements wherein the fatty acid ester is selected from glyceryl monooleate, glycerol palmitoleate, glycerol 1-stearate, 2-oleoylglycerol, glycerol monoisostearate, or mixtures thereof.
33. The hair treatment composition according to any of the preceding statements further comprising an additive for the secondary component, wherein the additive comprises at least one or more of a preformed polymer, a nonionic surfactant, a cationic surfactant, a coloring agent; a fatty alcohol ester composed of a linear, unsaturated or saturated C10-C30 fatty alcohol esterified with a C3-C6 mono or dicarboxylic acid, a linear, saturated or unsaturated C10-C30 fatty alcohol, a cationic polymer, aluminosilicate clay microparticles, silicate microparticles, a humectant, emulsifier, vegetable oil, petroleum jelly, liquid petrolatum, a silicone, paraffin, wax, beeswax, mineral oil and/or any combination thereof.
34. A hair treatment composition according to any of the preceding statements wherein the additive for the secondary component comprises at least a fatty alcohol ester.
35. The hair treatment composition according to any of preceding statements wherein the additive for the secondary component comprises at least the fatty alcohol ester wherein the fatty alcohol ester comprises the esterified fatty alcohol comprises a C10 - C30 saturated or unsaturated alcohol and the esterifying acid comprises a C3-C6 mono or dicarboxylic acid.
36. The hair treatment composition according to any of the preceding statements comprising the primary component of cosmetically acceptable medium and combination of a C3-C6 linear saturated dicarboxylic acid with one hydroxyl substituent and a fatty C16-C20 linear unsaturated monocarboxylic acid with one olefinic unsaturation.
37. The hair treatment composition according to any of the preceding statements further comprising the secondary component comprising at least a C10-C30 saturated branched hydrocarbon, and a fatty acid ester wherein the esterifying alcohol is a C3-C6 triol, the esterified fatty acid is the fatty C16-C20 linear unsaturated monocarboxylic acid and the triol is monoesterified with the fatty acid.
38. The hair treatment composition according to any of the preceding statements wherein the primary component comprises malic acid, and oleic acid or palmitoleic acid, preferably oleic acid.
39. The hair treatment composition according to any of the preceding statements wherein the secondary component comprises at least a hydrocarbon selected from the group consisting of:
   c1) a C10 to C40 linear saturated hydrocarbon;
   c2) a C10 to C40 linear unsaturated hydrocarbon with from 1 to 6 olefinic unsaturations;
   c3) a C10 to C40 branched saturated hydrocarbon;
   c4) a C10 to C40 branched unsaturated hydrocarbon with from 1 to 6 olefinic unsaturations;
   c6) a C20 to C30 linear saturated hydrocarbon;
   c7) a C20 to C30 linear unsaturated hydrocarbon with from 1 to 6 olefinic unsaturations;
   c8) a C20 to C30 branched saturated hydrocarbon;
   c9) a C20 to C30 branched unsaturated hydrocarbon with from 1 to 6 olefinic unsaturations;
   c10) liquid mineral oil;
   c11) liquid paraffin;
   c12) any combination thereof, and,
   c13) preferably the hydrocarbon is liquid paraffin, a saturated C16-C18 branched or linear hydrocarbon, a saturated or unsaturated diterpene (C20), a saturated or unsaturated triterpene (C30), squalene or squalane.
40. The hair treatment composition according to any of the preceding statements further comprising the additive of the secondary component comprising at least a saturated or unsaturated C10-C30 fatty alcohol.
41. The hair treatment composition according to any of the preceding statements further comprising the additive of the secondary component comprising at least the saturated or unsaturated fatty alcohol comprising cetyl alcohol, stearyl alcohol, palmityl alcohol or any combination thereof.
42. The hair treatment composition according to any of the preceding statements further comprising the additive for the a secondary component comprising at least a cationic surfactant and/or a silicone additive.
43. The hair treatment composition according to any of the preceding statements further comprising the additive for the a secondary component comprising at least a coloring agent.
44. The hair treatment composition according to any of the preceding statements further comprising the additive for the secondary component comprising at least a cationic polymer.
45. The hair treatment composition according to any of the preceding statements further comprising the additive for the secondary component comprising at least a nonionic surfactant.
46. The hair treatment composition according to any of the preceding statements further comprising the additive for the secondary component comprising at least a preformed polymer.
47. The hair treatment composition according to any of the preceding statements further comprising the additive for the secondary component comprising at least silicate and/or aluminosilicate clay microparticles.
48. The hair treatment composition according to any of the preceding statements wherein the primary component comprises the dicarboxylic acid at a concentration range of from about 0.1 wt% to about 20 wt%.
49. The hair treatment composition according to any of the preceding statements wherein the primary component comprises the fatty monocarboxylic acid at a concentration a range of about 0.015 wt% to about 20 wt%.
50. The hair treatment composition according to any of the preceding statements comprising the secondary component at a concentration range a range of about 0.01 wt% to about 40 wt%, preferably up to about 35 wt%, more preferably up to about 30 wt%, especially more preferably up to about 25 wt%, most preferably up to about 20 wt%, especially most preferably up to about 15 wt% relative to the total weight of the composition.
51. The hair treatment composition according to any of the preceding statements wherein the primary component comprises a wt percentage ratio of dicarboxylic acid to fatty monocarboxylic acid comprises about 30:1 to about 1:5, preferably about 30:1 to about 1:3, more preferably about 30:1 to about 1:1.75.
52. The hair treatment composition according to any of the preceding statements wherein the wt percentage ratio of the wt percentage of primary component to the wt percentage of the secondary component comprises about 20:1 to about 1:20, preferably about 10:1 to about 1: 10, more preferably about 5:1 to about 1:5, especially about 4:1 to about 1:4 including all ratios therebetween.
53. The hair treatment composition according to any of the preceding statements wherein the medium comprises an aqueous or aqueous organic medium.
53. The hair treatment composition according to any of the preceding statements wherein the medium comprises an aqueous organic medium and the organic portion comprises at least a C2-C6 linear or branched alcohol.
55. The hair treatment composition according to any of the preceding statements, wherein the composition further comprises additives to provide a hair cream, hair rinse, hair oil, hair pomade, hair foam, hair spray, hair tonic and/or hair lotion.
56. Use of the hair treatment composition according to any of the preceding statements as a general stand-alone conditioner, a stand-alone leave-on conditioner, a stand-alone rinse-off conditioner or a stand-alone mask.
57. Use of the hair treatment composition according to any of the preceding statements wherein any of one or more of the stand-alone conditioners and the stand-alone mask is formulated as a hair gel, hair paste, hair creme, hair rinse, hair oil, hair pomade, hair foam, hair spray, hair aerosol, hair tonic and/or hair lotion.
58. A method for conditioning anagenic hair comprising applying to the hair the hair treatment composition of any of the preceding statements.
59. The method according to any of the preceding method statements wherein the hair treatment composition is diluted with water before or while applying to the hair.
60. The method according to any of the preceding method statements wherein the hair treatment composition is applied to the hair without dilution with water or other medium.
61. A cleaning and conditioning regimen for hair comprising as a first step, shampooing the hair with a cleansing and cleaning composition comprising a sulfate and/or a non-sulfate anionic detergent in an aqueous organic medium, rinsing the hair to remove the cleansing and cleaning composition, optionally drying the hair to dampness and as a second step applying a hair treatment composition of any of the preceding statements 1-55.
62. The cleaning and conditioning regimen for hair according to statement 61 wherein the hair is rinsed or toweled or combed or any combination thereof after the second step.
63. The cleaning and conditioning regimen for hair according to statement 61 or 62 wherein the cleansing and cleaning composition is a gel, crème, lotion, oil, aerosol, spray or flowable composition.
64. A kit comprising a contained unit of cleansing and cleaning composition of a sulfate and/or non-sulfate anionic detergent in an aqueous organic medium and a contained unit of the hair treatment composition of any of the preceding statements 1-55.
65. The kit according to statement 64 wherein the cleansing and cleaning composition is is a gel, crème, lotion, oil, aerosol, spray or flowable composition.

### MISCELLANEOUS STATEMENTS

The invention has been described broadly and generically herein. Each of the narrower species and subgeneric groupings falling within the generic disclosure also form part of the invention. This includes the generic description of the invention with a proviso or negative limitation removing any matter from the genus, regardless of whether or not the excised material is specifically recited herein. The inventions, examples, results and statement of embodiments described, stated and claimed herein may have attributes and embodiments include, but not limited to, those set forth or described or referenced in this application.

The terms and expressions that have been employed are used as terms of description and not of limitation, and there is no intent in the use of such terms and expressions to exclude any equivalent of the features shown and described or portions thereof, but it is recognized that various modifications are possible within the scope of the invention as claimed and as provided by the statements of embodiments. Thus, it will be understood that although the present invention has been specifically disclosed by various nonlimiting embodiments and/or preferred nonlimiting embodiments and optional features, any and all modifications and variations of the concepts herein disclosed that may be resorted to by those skilled in the art are considered to be within the scope of this invention as defined by the appended claims and the statements of embodiments.

All patents, publications, scientific articles, web sites and other documents and ministerial references or mentioned herein are indicative of the levels of skill of those skilled in the art to which the invention pertains, and each such referenced document and material is hereby incorporated by reference to the same extent as if it had been incorporated verbatim and set forth in its entirety herein. The right is reserved to physically incorporate into this specification any and all materials and information from any such patent, publication, scientific article, web site, electronically available information, textbook or other referenced material or document.

The written description of this patent application includes all claims, examples and statements of embodiments. All claims and statements of embodiments including all original claims are hereby incorporated by reference in their entirety into the written description portion of the specification and the right is reserved to physically incorporate into the written description or any other portion of the application any and all such claims and statements of embodiments. Thus, for example, under no circumstances may the patent be interpreted as allegedly not providing a written description for a claim on the assertion that the precise wording of the claim is not set forth in haec verba in written description portion of the patent.

While the invention has been described in conjunction with the detailed description thereof, the foregoing description is intended to illustrate and not limit the scope of the invention, which is defined by the scope of the appended claims and the statements of embodiments. Thus, from the foregoing, it will be appreciated that, although specific nonlimiting embodiments of the invention have been described herein for the purpose of illustration, various modifications may be made without deviating from the scope of the invention. Other aspects, advantages, and modifications are within the scope of the following claims and the present invention is not limited except as by the appended claims and the statements of embodiments.

The specific methods and compositions described herein are representative of preferred nonlimiting embodiments and are exemplary and not intended as limitations on the scope of the invention. Other objects, aspects, and embodiments will occur to those skilled in the art upon consideration of this specification and are encompassed within the spirit of the invention as defined by the scope of the claims. It will be readily apparent to one skilled in the art that varying substitutions and modifications may be made to the invention disclosed herein without departing from the scope and spirit of the invention. The invention illustratively described herein suitably may be practiced in the absence of any element or elements, or limitation or limitations, which is not specifically disclosed herein as essential. Thus, for example, in each instance herein, in nonlimiting embodiments or examples of the present invention, the terms "comprising", "including", "containing", "providing" and the like are to be read expansively and without limitation.

The methods and processes illustratively described herein suitably may be practiced in differing orders of steps, and that they are not necessarily restricted to the orders of steps indicated herein or in the claims.

## Claims

1. A hair treatment composition comprising a general stand-alone conditioner, a stand-alone leave-on conditioner, a stand-alone rinse-off conditioner or a stand-alone mask comprising a cosmetically acceptable aqueous or aqueous-organic medium and at least the primary component comprising:
a) a linear, saturated C3-C6 dicarboxylic acid substituted by one hydroxyl group; and
b) a linear mono-unsaturated C16-C18 fatty monocarboxylic acid; and/or,
c) the cosmetically acceptable salts of the dicarboxylic acid and/or the fatty monocarboxylic acid; wherein,
the concentration range of the dicarboxylic acid comprises about 0.1 wt percent to about 15 wt percent,
the concentration range of the fatty monocarboxylic acid comprises about 0.015 wt percent to about 15 wt percent;
the wt percentage ratio of dicarboxylic acid to fatty monocarboxylic acid comprises about 30:1 to about 1:5 preferably about 30:1 to about 1:3 more preferably about 30:1 to about 1:1.75; and,
the concentrations of the dicarboxylic acid and the fatty monocarboxylic acid are relative to the total weight of the composition.

2. The hair treatment composition according to any of the preceding claims further comprising a secondary component comprising:
a) a fatty acid ester composed of a linear, unsaturated or saturated C10-C30 fatty acid esterified with a C2-C6 mono-, di-, or tri hydroxyl alcohol; and
b) a C10-C30 saturated and/or unsaturated, linear and/or branched hydrocarbon;
wherein the secondary component is present at a weight percentage concentration comprising from about 0.01 wt% to about 40 wt%, more preferably up to about 35 wt%, most preferably up to about 30 wt%, especially most preferably up to about 20 wt% relative to the total weight of the composition.

3. The hair treatment composition according to any of the preceding claims wherein
a) the dicarboxylic acid comprises malic acid, hydroxysuccinic acid or hydroxyglutaric acid, or salts thereof or any combination thereof; preferably malic acid or hydroxysuccinic acid or salts thereof, or any combination thereof; more preferably malic acid and/or a salt thereof; and
b) the fatty monocarboxylic acid comprises palmitoleic acid, sapienic acid, oleic acid or elaidic acid or salts thereof, or any combination thereof; preferably palmitoleic acid, sapienic acid or oleic acid or salts thereof or any combination thereof; more preferably oleic acid and/or a salt thereof.

4. The hair treatment composition according to any of the preceding claims wherein the fatty acid ester of the secondary component comprises the fatty monocarboxylic acid esterified by a C1 to C10 mono alcohol, a C2 to C10 diol and/or a C3 to C10 triol, preferably wherein the esterifying alcohol is a C3-C6 triol, or more preferably wherein the esterified fatty acid is a fatty C16-C20 linear unsaturated monocarboxylic acid and the C3-C6 triol is monoesterified with the fatty acid; and the weight percentage concentration of the fatty acid ester comprises a range from about 0.1 wt% to about 10 wt% relative to the total weight of the composition.

5. The hair treatment composition according to any of the preceding claims comprising the secondary component comprising one or more of liquid paraffin, isohexadecane, squalene or squalane, glyceryl or isopropyl oleate, or glyceryl or isopropyl palmitoleate, or glyceryl or isopropyl myristate or any combination thereof.

6. The hair treatment composition according to any of the preceding claims further comprising an additive for the a secondary component wherein the additive comprises at least one or more of a fatty alcohol ester composed of a linear, unsaturated or saturated C10-C30 fatty alcohol esterified with a C3-C6 mono or dicarboxylic acid; a linear, saturated or unsaturated C10-C30 fatty alcohol; a cationic surfactant, a cationic polymer, a nonionic surfactant, a rheologic control agent, a silicone, a preformed polymer, an acrylate polymer selected from at least a polyacrylic ester, a polyacrylic amide and/or an acrylates copolymer, a coloring agent, an aluminosilicate as microparticles, a silicate as microparticles, a suspending agent, humectant, emulsifier, vegetable oil, petroleum jelly, liquid petrolatum, paraffin, wax, beeswax, mineral oil and/or any combination thereof, wherein,
the additive for the secondary component is present at a weight percentage concentration comprising from about 0.01 wt% to about 35 wt%, preferably up to about 25 wt%, more preferably up to about 20 wt%, especially more preferably up to about 15 wt% relative to the total weight of the composition.

7. The hair treatment composition according to any of the preceding claims comprising the additive for the secondary component comprising one or more of behentrimonium chloride, cetrimonium chloride, ceteareth-15 to -25, steareth-15 to 25, stearyl alcohol, cetyl alcohol, acrylates copolymer, polyacrylamide, poly (methyl/ethyl (meth)acrylate), amodimethicone, or any combination thereof.

8. The hair treatment composition according to any of the preceding claims wherein the dicarboxylic acid of the primary component is malic acid, and/or the salt thereof; and/or the fatty monocarboxylic acid of the primary component is oleic acid and/or the salt thereof.

9. The hair treatment composition according to any of the preceding claims, wherein the general stand-alone conditioner, stand-alone leave-on conditioner, stand-alone rinse-off conditioner or stand-alone mask comprises additives to provide a hair gel, hair paste, hair creme, hair rinse, hair liquid, hair oil, hair pomade, hair mousse, hair foam, hair spray, hair aerosol, hair tonic and/or hair lotion.

10. A method for conditioning hair comprising applying to the hair a hair treatment composition comprising the general stand-alone conditioner, the stand-alone leave-on conditioner, the stand-alone rinse-off conditioner or the stand-alone mask according to any of claims 1-9.

11. A cleaning and conditioning regimen for hair comprising as a first step, shampooing the hair with a cleansing and cleaning composition comprising a sulfate and/or a non-sulfate anionic detergent in an aqueous organic medium; rinsing the hair to remove the cleansing and cleaning composition; optionally drying the hair to dampness; and as a second step applying to the hair a hair treatment composition of any of the preceding claims 1-9; and thereafter optionally rinsing and/or toweling and/or combing the hair.

12. A kit comprising a contained unit of a cleansing and cleaning composition comprising a sulfate and/or non-sulfate anionic detergent in an aqueous or aqueous-organic medium and a contained unit of the hair treatment composition of any of the claims 1-9.

13. The kit according to claim 12 wherein the cleansing and cleaning composition further comprises a cationic surfactant.

14. The kit according to claim 13 wherein the cleansing and cleaning composition includes a coacervate complex of the cationic surfactant and the anionic surfactant.

15. Use of the hair treatment composition according to any of the preceding claims 1-9 as a general stand-alone conditioner, a stand-alone leave-on conditioner, a stand-alone rinse-off conditioner or a stand-alone mask or as the second unit of a two unit kit of a first unit of a cleansing and cleaning composition unit and a second unit of general stand-alone conditioner, a stand-alone leave-on conditioner or a stand-alone rinse-off conditioner.
